(19) Europäisches Patentamt / European Patent Office / Office européen des brevets

(11) **EP 4 782 009 A1**

(12) **EUROPEAN PATENT APPLICATION**
published in accordance with Art. 153(4) EPC


(43) Date of publication:
**29.07.2026 Bulletin 2026/31**

(21) Application number: **24867515.9**

(22) Date of filing: **19.09.2024**

(51) International Patent Classification (IPC):
***A61K 47/68*** *(2017.01)* ***A61K 39/00*** *(2006.01)*
***C07K 16/28*** *(2006.01)* ***A61P 35/00*** *(2006.01)*

(52) Cooperative Patent Classification (CPC):
**A61K 39/00; A61K 47/68; A61P 35/00; C07K 16/28**

(86) International application number:
**PCT/CN2024/119856**

(87) International publication number:
**WO 2025/061110 (27.03.2025 Gazette 2025/13)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **19.09.2023 CN 202311206601**
**24.08.2024 CN 202411170320**



(71) Applicant: **Medilink Therapeutics (Suzhou) Co., Ltd.**
**Suzhou, Jiangsu 215000 (CN)**

(72) Inventors:
- **XUE, Tongtong**
  **Suzhou, Jiangsu 215000 (CN)**
- **ZHANG, Li**
  **Suzhou, Jiangsu 215000 (CN)**
- **ZHANG, Xian**
  **Suzhou, Jiangsu 215000 (CN)**
- **CHIN, Steve**
  **Suzhou, Jiangsu 215000 (CN)**
- **CAI, Jiaqiang**
  **Suzhou, Jiangsu 215000 (CN)**

(74) Representative: **Mewburn Ellis LLP**
**Aurora Building**
**Counterslip**
**Bristol BS1 6BX (GB)**


Remarks:
The complete document including Reference Table(s) and the Sequence Listing(s) can be downloaded from the EPO website

(54) **USE OF ANTI-B7H3 ANTIBODY-DRUG CONJUGATE IN PREPARATION OF DRUG FOR TREATING NASOPHARYNGEAL CARCINOMA AND/OR LYMPHOEPITHELIOMA-LIKE CARCINOMA**


(57) The present disclosure belongs to the technical field of pharmaceuticals. Specifically, the present invention relates to an anti-B7H3 antibody-drug conjugate, a pharmaceutically acceptable salt, stereoisomer, or metabolite thereof, or a solvate of any of the aforementioned items, and a use thereof in the preparation of a drug for treating nasopharyngeal carcinoma and/or lymphoepithelioma-like carcinoma.


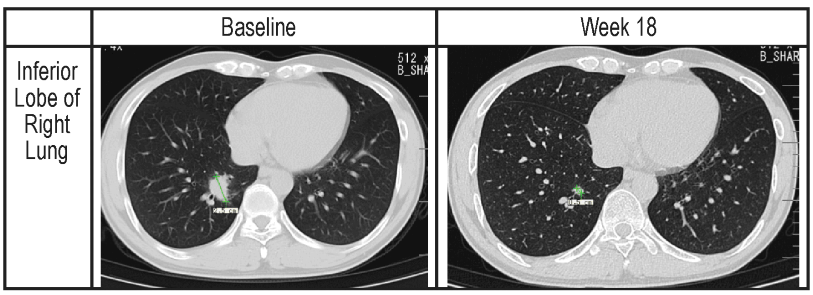


FIG. 3

EP 4 782 009 A1

## Description

This application claims priority of Chinese Patent Application 202311206601.3 filed on September 19, 2023 and priority of Chinese Patent Application 202411170320.1 filed on August 24, 2024. The present application incorporates the entire text of the above-mentioned Chinese patent applications by reference.

## Technical Field

The present disclosure belongs to the technical field of pharmaceuticals. More specifically, the present invention relates to a therapeutic agent of an anti-B7H3 antibody-drug conjugate for nasopharyngeal carcinoma and/or lymphoepithelioma-like carcinoma, and/or methods and uses of the anti-B7H3 antibody-drug conjugate in the preparation of a drug for treating nasopharyngeal carcinoma and/or lymphoepithelioma-like carcinoma.

## Background Art

Malignant tumors have become a major global public health problem, resulting in the death of nearly 10 million people in 2020. The number of cancer patients worldwide and the number of people who die from cancer continue to rise, causing the overall market size of cancer treatment to continuously expand, and the demand for new therapeutic drugs and new treatment means continues to increase.

The nasopharyngeal carcinoma (NPC) refers to a malignant tumor occurring at an apex and lateral walls of a pharyngonasal cavity, with an incidence rate ranking the first among malignant tumors in otorhinolaryngology. The World Health Organization classifies nasopharyngeal carcinomas into three main pathological subtypes: keratinizing squamous cell carcinoma, non-keratinizing carcinoma (including a differentiated type and an undifferentiated type), and basaloid squamous cell carcinoma. The non-keratinizing carcinoma accounts for the majority of nasopharyngeal carcinomas and is closely associated with Epstein-Barr virus (EBV) infection.

Most nasopharyngeal carcinomas are sensitive to radiotherapy; therefore, radiotherapy is the preferred treatment method for early-stage nasopharyngeal carcinomas. However, due to the complex anatomy of the nasopharynx and the deep and concealed pathogenic site, approximately 75% of patients have already reached advanced stages at initial diagnosis, for patients with later disease courses, high degrees of differentiation, or recurrence after radiotherapy, the therapeutic effect of radiotherapy is limited, the prognosis is extremely poor, and surgical resection and chemotherapy remain the most prominent treatment means. Currently, chemotherapy for the nasopharyngeal carcinoma lacks individualized specific treatment means and drugs, mainly uses small-molecule cytotoxic agents (such as platinum, paclitaxel, gemcitabine, 5-FU, and others) and PD-(L)1 inhibitors, and requires combination of multiple drugs to achieve efficacy. However, due to poor tolerance of patients to the aforementioned treatment means (caused by high drug toxicity), the therapeutic effect on the nasopharyngeal carcinoma is suboptimal, and the 5-year survival rate remains low. There is an urgent need to develop new therapeutic drugs.

The lymphoepithelioma-like carcinoma (LELC) belongs to a type of primary non-small cell lung cancer (NSCLC), which is classified into a category of other unclassified carcinomas in the 2015 version of the WHO lung cancer histological classification. Due to the lack of prospective studies, the optimal treatment regimen and survival outcomes remain unclear, and treatment of this disease is primarily empirical. The most commonly used chemotherapy regimens include cisplatin or carboplatin combined with 5-fluorouracil, paclitaxel, docetaxel, or gemcitabine. The aforementioned drugs all have disadvantages such as high toxicity, poor tolerance, and propensity for drug resistance, and there is a need to develop more therapeutic drugs and treatment means.

Monoclonal antibody drugs have advantages such as strong targeting ability, high specificity, and low incidence of adverse reactions, and an antibody-drug conjugate (ADC) is exactly a representative product thereof. Since ADCs combine the advantages of both the effectiveness of small-molecule drugs and the targeting ability of antibody drugs, etc., they can reduce the toxic and side effects of cytotoxic agents while improving the therapeutic effects of the drugs. Currently, there are currently 15 ADC drugs approved on the market worldwide that have demonstrated excellent clinical therapeutic effects and high safety against breast cancer, lung cancer, and gastrointestinal cancers, but there is no variety against the nasopharyngeal carcinoma and the lymphoepithelioma-like carcinoma, representing a high unmet clinical need.

B7H3 is a member of the B7 family and belongs to the type I transmembrane protein. B7H3 protein is not expressed or is expressed at very low levels in normal tissues and cells, but is highly expressed in various tumor tissues, and is closely associated with tumor progression, patient survival, and prognosis. It has been reported (Wang YQ, et al. Development and validation of an immune checkpoint-based signature to predict prognosis in nasopharyngeal carcinoma using computational pathology analysis. J Immunother Cancer. 2019 Nov 13;7(1):298.) that B7H3 expression is present in more than 79% of nasopharyngeal carcinomas. In terms of pathological histomorphology, the lymphoepithelioma-like carcinoma is highly similar to the undifferentiated nasopharyngeal carcinoma.

## Summary of the Invention

In order to overcome the problems existing in the prior art, the present invention provides a use of an anti-B7H3 antibody-drug conjugate in the preparation of a drug for treating nasopharyngeal carcinoma and/or lymphoepithelioma-like carcinoma.

Antibody-Drug Conjugate

In some embodiments, the B7H3 antibody-drug conjugate is an antibody-drug conjugate as shown in Formula III, a pharmaceutically acceptable salt, a stereoisomer, or metabolite thereof, or a solvate of any of the aforementioned items, the antibody-drug conjugate as shown in Formula III being as follows,

$$Tb\text{-}[L\text{-}D]_q \qquad \text{Formula III}$$

wherein:

Tb is an anti-B7H3 antibody or an antigen-binding fragment thereof;
L is a linker;
D is a bioactive molecule fragment;
q is a drug-antibody conjugate ratio, and is selected from any numerical value between 0.1 and 16.0; in a preferred embodiment, q is selected from any integer between 1 and 10.

In some embodiments, q is a drug-antibody conjugate ratio, and is selected from any numerical value between 0.1 and 16.0, including both endpoint values.

In some embodiments, Tb is an anti-B7H3 antibody or an antigen-binding fragment thereof, which is an antibody numbered 1D1, 1D1-01, 2E3, 2E3-02 in WO2022170971, enoblituzumab, mirzotamab, omburtamab, or antigen-binding fragments thereof.

In some embodiments, the anti-B7H3 antibody or an antigen-binding fragment thereof comprises a heavy chain variable region and a light chain variable region, wherein:

the heavy chain variable region comprises:

(i) CDR-H1, which comprises an amino acid sequence as set forth in SEQ ID NO: 1;
(ii) CDR-H2, which comprises an amino acid sequence as set forth in SEQ ID NO: 2; and
(iii) CDR-H3, which comprises an amino acid sequence as set forth in SEQ ID NO: 3; and

the light chain variable region comprises:

(i) CDR-L1, which comprises an amino acid sequence as set forth in SEQ ID NO: 4;
(ii) CDR-L2, which comprises an amino acid sequence as set forth in SEQ ID NO: 5; and
(iii) CDR-L3, which comprises an amino acid sequence as set forth in SEQ ID NO: 6,

wherein CDRs are defined according to the Kabat numbering system.

In some embodiments, the heavy chain variable region of the anti-B7H3 antibody or the antigen-binding fragment thereof comprises an amino acid sequence as set forth in SEQ ID NO: 7 or having at least 85% sequence identity to SEQ ID NO: 7, and the light chain variable region comprises an amino acid sequence as set forth in SEQ ID NO: 8 or having at least 85% sequence identity to SEQ ID NO: 8.

In some embodiments, the heavy chain variable region of the anti-B7H3 antibody or the antigen-binding fragment thereof comprises an amino acid sequence having at least 90%, at least 91%, at least 92%, at least 93%, at least 94%, at least 95%, at least 96%, at least 97%, at least 98%, or at least 99% sequence identity to SEQ ID NO: 7, and the light chain variable region comprises an amino acid sequence having at least 90%, at least 91%, at least 92%, at least 93%, at least 94%, at least 95%, at least 96%, at least 97%, at least 98%, or at least 99% sequence identity to SEQ ID NO: 8.

In some embodiments, the heavy chain variable region of the anti-B7H3 antibody or the antigen-binding fragment thereof comprises an amino acid sequence as set forth in SEQ ID NO: 7, and the light chain variable region comprises an amino acid sequence as set forth in SEQ ID NO: 8.

In some embodiments, the heavy chain of the anti-B7H3 antibody comprises an amino acid sequence having at least 85%, at least 90%, at least 91%, at least 92%, at least 93%, at least 94%, at least 95%, at least 96%, at least 97%, at

least 98%, or at least 99% sequence identity to SEQ ID NO: 9, and the light chain comprises an amino acid sequence having at least 85%, at least 90%, at least 91%, at least 92%, at least 93%, at least 94%, at least 95%, at least 96%, at least 97%, at least 98%, or at least 99% sequence identity to SEQ ID NO: 10.

In some embodiments, the anti-B7H3 antibody comprises a heavy chain having an amino acid sequence as set forth in SEQ ID NO: 9, and a light chain having an amino acid sequence as set forth in SEQ ID NO: 10.

In some embodiments, the anti-B7H3 antibody-drug conjugate is an antibody-drug conjugate as shown in Formula I, a pharmaceutically acceptable salt, stereoisomer, or metabolite thereof, or a solvate of any of the aforementioned items, with a structure of Formula I as follows:

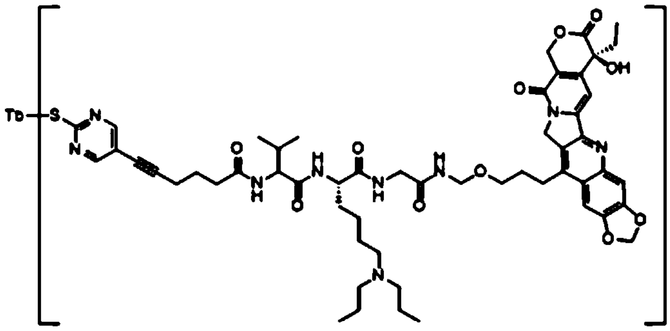

Formula I

wherein Tb is an anti-B7H3 antibody or an antigen-binding fragment thereof as described above;
S is a sulfur atom on Tb;
q is a drug-antibody conjugate ratio, and is selected from any numerical value between 0.1 and 16.0, preferably 1 to 10; more preferably, q is 1, 2, 3, 4, 5, 6, 7, or 8; most preferably, q is 2, 4, 6, or 8.

In some embodiments, the anti-B7H3 antibody-drug conjugate is an antibody-drug conjugate as shown in Formula II, a pharmaceutically acceptable salt, stereoisomer, or metabolite thereof, or a solvate of any of the aforementioned items, with a structure of Formula II as follows:

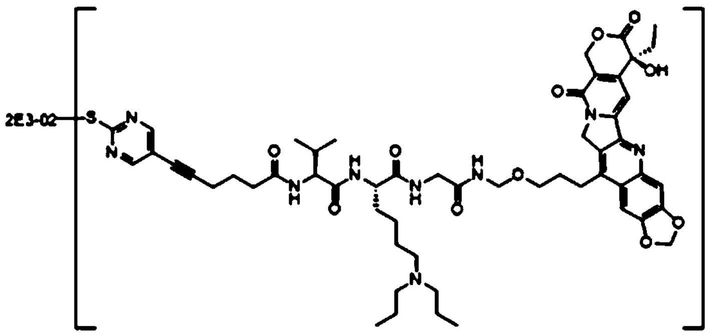

Formula II.

Therapeutic Uses and Methods

In some embodiments, the present invention provides a use of an anti-B7H3 antibody-drug conjugate (preferably, Formula II), a pharmaceutically acceptable salt, stereoisomer, or metabolite thereof, or a solvate of any of the aforementioned items in the preparation of a drug for treating nasopharyngeal carcinoma, wherein the nasopharyngeal carcinoma includes but is not limited to keratinizing squamous cell carcinoma, non-keratinizing nasopharyngeal carcinoma, and basaloid squamous cell carcinoma.

In some embodiments, the present invention provides a use of an anti-B7H3 antibody-drug conjugate (preferably, Formula II), a pharmaceutically acceptable salt, stereoisomer, or metabolite thereof, or a solvate of any of the aforementioned items in the preparation of a drug for treating non-keratinizing nasopharyngeal carcinoma, wherein the non-keratinizing nasopharyngeal carcinoma includes a differentiated type or an undifferentiated type.

In some embodiments, the present invention provides a use of an anti-B7H3 antibody-drug conjugate (preferably, Formula II), a pharmaceutically acceptable salt, stereoisomer, or metabolite thereof, or a solvate of any of the aforementioned items in the preparation of a drug for treating in situ nasopharyngeal carcinoma or infiltrative nasopharyngeal carcinoma. Further, the infiltrative nasopharyngeal carcinoma includes but is not limited to squamous cell carcinoma, adenocarcinoma, microinvasive carcinoma, vesicular cell carcinoma, and undifferentiated nasopharyngeal carcinoma.

In some embodiments, the present invention provides a use of an anti-B7H3 antibody-drug conjugate (preferably, Formula II), a pharmaceutically acceptable salt, stereoisomer, or metabolite thereof, or a solvate of any of the aforementioned items in the preparation of a drug for treating recurrent and/or advanced and/or metastatic nasopharyngeal carcinoma.

In some embodiments, provided is a method of treating keratinizing squamous cell carcinoma of nasopharynx, and the method comprises administering to a patient in need of treatment a therapeutically effective amount of an anti-B7H3 antibody-drug conjugate (preferably, Formula II), a pharmaceutically acceptable salt, stereoisomer, or metabolite thereof, or a solvate of any of the aforementioned items.

In some embodiments, provided is a method of treating non-keratinizing nasopharyngeal carcinoma, the non-keratinizing nasopharyngeal carcinoma includes a differentiated type or an undifferentiated type, and the method comprises administering to a patient in need of treatment a therapeutically effective amount of an anti-B7H3 antibody-drug conjugate (preferably, Formula II), a pharmaceutically acceptable salt, stereoisomer, or metabolite thereof, or a solvate of any of the aforementioned items.

In some embodiments, provided is a method of treating basaloid squamous cell carcinoma of nasopharynx, and the method comprises administering to a patient in need of treatment a therapeutically effective amount of an anti-B7H3 antibody-drug conjugate (preferably, Formula II), a pharmaceutically acceptable salt, stereoisomer, or metabolite thereof, or a solvate of any of the aforementioned items.

In some embodiments, provided is a method of treating in situ nasopharyngeal carcinoma, and the method comprises administering to a patient in need of treatment a therapeutically effective amount of an anti-B7H3 antibody-drug conjugate (preferably, Formula II), a pharmaceutically acceptable salt, stereoisomer, or metabolite thereof, or a solvate of any of the aforementioned items.

In some embodiments, provided is a method of treating infiltrative nasopharyngeal carcinoma, the infiltrative nasopharyngeal carcinoma includes but is not limited to squamous cell carcinoma, adenocarcinoma, microinvasive carcinoma, vesicular cell carcinoma, and undifferentiated nasopharyngeal carcinoma, and the method comprises administering to a patient in need of treatment a therapeutically effective amount of an anti-B7H3 antibody-drug conjugate (preferably, Formula II), a pharmaceutically acceptable salt, stereoisomer, or metabolite thereof, or a solvate of any of the aforementioned items.

In some embodiments, provided is a method of treating recurrent and/or advanced and/or metastatic nasopharyngeal carcinoma, and the method comprises administering to a patient a therapeutically effective amount of an anti-B7H3 antibody-drug conjugate (preferably, Formula II), a pharmaceutically acceptable salt, stereoisomer, or metabolite thereof, or a solvate of any of the aforementioned items.

In some embodiments, the nasopharyngeal carcinoma and/or lymphoepithelioma-like carcinoma is not surgically resectable.

In some embodiments, a patient with the nasopharyngeal carcinoma and/or lymphoepithelioma-like carcinoma has previously received chemotherapy and/or monoclonal antibody treatment and/or radiotherapy.

In some embodiments, a patient with the nasopharyngeal carcinoma and/or lymphoepithelioma-like carcinoma has disease progression after previously receiving chemotherapy and/or monoclonal antibody treatment and/or radiotherapy.

In some embodiments, a patient with metastatic or recurrent nasopharyngeal carcinoma refers to a patient who has previously received platinum-containing chemotherapy treatment and has recorded disease progression or intolerance during or after treatment.

In some embodiments, the patient with the metastatic or recurrent nasopharyngeal carcinoma refers to a patient who has previously received at least second-line (at least platinum-containing) chemotherapy treatment and has recorded disease progression or intolerance during or after the treatment.

In some embodiments, the patient with the metastatic or recurrent nasopharyngeal carcinoma refers to a patient who has previously received at least second-line (at least first-line platinum-containing) chemotherapy treatment and has recorded disease progression or intolerance during or after the treatment.

In some embodiments, a patient with metastatic or recurrent nasopharyngeal carcinoma refers to a patient who has previously received anti-PD-1 drug and/or anti-PD-L1 drug treatment and has recorded disease progression or intolerance during or after the treatment.

In some embodiments, the patient with the metastatic or recurrent nasopharyngeal carcinoma refers to a patient who has previously received anti-PD-1 drug treatment and has recorded disease progression or intolerance during or after the treatment.

In some embodiments, the patient with the metastatic or recurrent nasopharyngeal carcinoma refers to a patient who has previously received anti-PD-L1 drug treatment and has recorded disease progression or intolerance during or after the treatment.

In some embodiments, a patient with metastatic or recurrent nasopharyngeal carcinoma refers to a patient who has previously received at least second-line (received at least platinum-containing) chemotherapy, anti-PD-1 drug and/or anti-PD-L1 drug treatment and has recorded disease progression or intolerance during or after the treatment.

In some embodiments, the patient with the metastatic or recurrent nasopharyngeal carcinoma refers to a patient who has previously received platinum-containing chemotherapy and anti-PD-1 drug treatment and has recorded disease progression or intolerance during or after the treatment.

In some embodiments, the patient with the metastatic or recurrent nasopharyngeal carcinoma refers to a patient who has previously received platinum-containing chemotherapy and anti-PD-L1 drug treatment and has recorded disease progression or intolerance during or after the treatment.

In some embodiments, the patient with the metastatic or recurrent nasopharyngeal carcinoma refers to a patient who has previously received at least second-line (received at least platinum-containing) chemotherapy and anti-PD-(L)1 treatment and has recorded disease progression or intolerance during or after the treatment.

In some embodiments, a patient with the metastatic or recurrent nasopharyngeal carcinoma refers to a patient who has previously received at least platinum-containing chemotherapy, wherein the platinum includes but is not limited to: cisplatin, carboplatin, nedaplatin, carboquone, and/or lobaplatin (oxaliplatin), or combinations thereof.

In some embodiments, the patient with the metastatic or recurrent nasopharyngeal carcinoma has previously received an anti-PD1 drug therapeutic agent, including: AK103 (HX008), treprinumab, sintilimab, camrelizumab, tislelizumab, nivolumab (Opdivo or Nivolumab), pembrolizumab (Keytruda or Pembrolizumab), nofazinlimab, serplulimab (Serplulimab, HLX10 or Hansizhuang), geptanolimab, Lipustobart, BAT-1306, Finotonlimab, Rulonilimab, SG001, Zimberelimab, Spartalizumab, Cemiplimab, STI-A1110, or one or more the aforementioned items.

In some embodiments, the patient with the metastatic or recurrent nasopharyngeal carcinoma has previously received an anti-PDL1 drug therapeutic agent, including: durvalumab, atezolizumab, envafolimab, sugemalimab, adebrelimab, tagitanlimab, socazolimab, benmelstobart, or combinations thereof.

In some embodiments, the patient with the nasopharyngeal carcinoma and/or lymphoepithelioma-like carcinoma has previously received one or more of the following chemotherapeutic agents, including gemcitabine, capecitabine, cisplatin, lobaplatin, nedaplatin, fluorouracil, paclitaxel, albumin-bound paclitaxel, docetaxel, irinotecan, vinorelbine, cyclophosphamide, and pemetrexed.

In some embodiments, the patient with the nasopharyngeal carcinoma and/or lymphoepithelioma-like carcinoma has previously received one or more of the following chemotherapeutic agents, including gemcitabine, capecitabine, cisplatin, lobaplatin, nedaplatin, fluorouracil, paclitaxel, albumin-bound paclitaxel, docetaxel, vinorelbine, cyclophosphamide, and pemetrexed.

In some embodiments, a patient with the nasopharyngeal carcinoma has previously received monoclonal antibody therapeutic agents, including camrelizumab, treprinumab, nivolumab, pembrolizumab, serplulimab, tislelizumab, cetuximab, or bevacizumab.

In some embodiments, the treatment comprises administering to the patient a therapeutically effective amount of an anti-B7H3 antibody-drug conjugate, a pharmaceutically acceptable salt, stereoisomer, or metabolite thereof, or a solvate of any of the aforementioned items, wherein for the anti-B7H3 antibody-drug conjugate (preferably, Formula II), the effective amount is administered at a dose of 0.1-15 mg/kg body weight of an individual, and the dose is preferably 0.1-10 mg/kg, 0.2-8 mg/kg, 0.3-6 mg/kg, 0.4-4 mg/kg, 0.5-3.6 mg/kg, or 1-3 mg/kg, more preferably 0.4 mg/kg, 0.5 mg/kg, 0.6 mg/kg, 0.8 mg/kg, 1.0 mg/kg, 1.2 mg/kg, 1.4 mg/kg, 1.6 mg/kg, 1.8 mg/kg, 2.0 mg/kg, 2.1 mg/kg, 2.2 mg/kg, 2.3 mg/kg, 2.4 mg/kg, 2.5 mg/kg, 2.6 mg/kg, 2.7 mg/kg, 2.8 mg/kg, 2.9 mg/kg, 3.0 mg/kg, 3.2 mg/kg, 3.4 mg/kg, 3.6 mg/kg, 3.8 mg/kg, 4.0 mg/kg, or 5.0 mg/kg.

In some embodiments, the treatment comprises administering to the patient a therapeutically effective amount of an anti-B7H3 antibody-drug conjugate, a pharmaceutically acceptable salt, stereoisomer, or metabolite thereof, or a solvate of any of the aforementioned items, wherein for the anti-B7H3 antibody-drug conjugate (preferably, Formula II), the effective amount is administered at a dose of 0.8-2.4 mg/kg body weight of an individual, preferably 1.6-2.4 mg/kg or 2.0-2.4 mg/kg, more preferably 1.6 mg/kg, 2.0 mg/kg, or 2.4 mg/kg.

In some embodiments, the routes of administration can be oral administration, parenteral administration, or transdermal administration; the parenteral administration includes but is not limited to intravenous injection, subcutaneous injection, or intramuscular injection; further, the route of administration is preferably intravenous injection.

Among them, an injection form comprises an injection solution or a lyophilized powder, which contains an anti-B7H3 antibody-drug conjugate (preferably, Formula II), a pharmaceutically acceptable salt, stereoisomer, or metabolite thereof, or a solvate of any of the aforementioned items, and optionally a buffer, a stabilizer, a pH adjusting agent, and/or a surfactant, wherein the buffer may be selected from one or more of acetate, citrate, succinate, and phosphate; the stabilizer may be selected from sugar or amino acid, preferably disaccharide, such as sucrose, lactose, trehalose, or

maltose; the pH adjusting agent may be selected from one or more of sodium hydroxide, lithium hydroxide, and potassium hydroxide; the surfactant may be selected from one or more of polyoxyethylene hydrogenated castor oil, glycerol fatty acid ester, and polyoxyethylene sorbitan fatty acid ester; preferably, the polyoxyethylene sorbitan fatty acid ester is one or more of polysorbate 20, 40, 60, or 80, most preferably polysorbate 20.

In some embodiments, a dosing frequency is once daily, once weekly, once every two weeks, once every three weeks, once every four weeks or once monthly, once every five weeks, once every six weeks, or twice every two weeks, twice every three weeks, twice every four weeks or twice monthly, twice every five weeks, twice every six weeks.

In some embodiments, the dosing frequency is preferably once every three weeks or twice every three weeks.

In some embodiments, a dosing cycle is one week, two weeks, three weeks, one month, two months, three months, four months, five months, six months, or longer; optionally, a duration of each dosing cycle is the same or different, and an interval between each dosing cycle is the same or different.

In some embodiments, the present invention further determines a B7H3 expression level in tumor tissues or a level of soluble B7H3 in peripheral blood, or detects biomarkers related to therapeutic efficacy, and is used for guiding the administration of the drug through studying the correlation between these biomarkers and the therapeutic effect of the anti-B7H3 antibody-drug conjugate.

In another aspect, the present invention provides an anti-B7H3 antibody-drug conjugate, a pharmaceutically acceptable salt, stereoisomer, metabolite thereof, or a solvate for use in a method of treating cancer, wherein the method of treating cancer is by administering to a subject in need thereof with nasopharyngeal carcinoma and/or lymphoepithelioma-like carcinoma the anti-B7H3 antibody-drug conjugate, and the anti-B7H3 antibody-drug conjugate has a structural formula I:

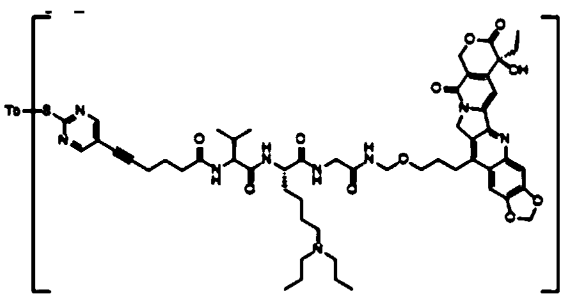

Formula I

wherein:

Tb is an anti-B7H3 antibody or an antigen-binding fragment thereof;
S is a sulfur atom on Tb;
q is any numerical value between 1 and 16, including both end values.

In some embodiments, the anti-B7H3 antibody-drug conjugate, or the pharmaceutically acceptable salt, stereoisomer, metabolite thereof, or the solvate of any of the aforementioned items, wherein in the method of treating cancer, the anti-B7H3 antibody-drug conjugate is as described in any of the embodiments herein.

In another aspect, the present invention provides an anti-B7H3 antibody-drug conjugate, a pharmaceutically acceptable salt, stereoisomer, metabolite thereof, or a solvate of any of the aforementioned items for treating nasopharyngeal carcinoma and/or lymphoepithelioma-like carcinoma, the anti-B7H3 antibody-drug conjugate having a structural formula I:

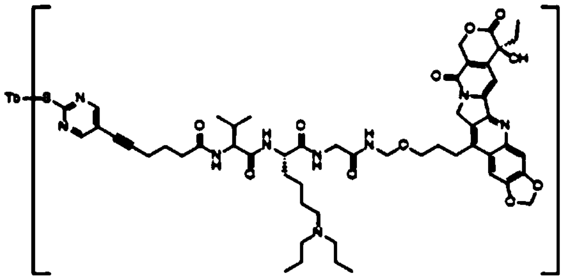

Formula I

wherein:

Tb is an anti-B7H3 antibody or an antigen-binding fragment thereof;
S is a sulfur atom on Tb;
q is any numerical value between 1 and 16, including both end values;
the nasopharyngeal carcinoma and the lymphoepithelioma-like carcinoma are as described above.

In another aspect, the present invention provides a method of treating nasopharyngeal carcinoma and/or lymphoepithelioma-like carcinoma, wherein the treatment comprises administering to a subject in need thereof an anti-B7H3 antibody-drug conjugate, a pharmaceutically acceptable salt, stereoisomer, metabolite thereof, or a solvate of any of the aforementioned items, the anti-B7H3 antibody-drug conjugate having a structural formula I:

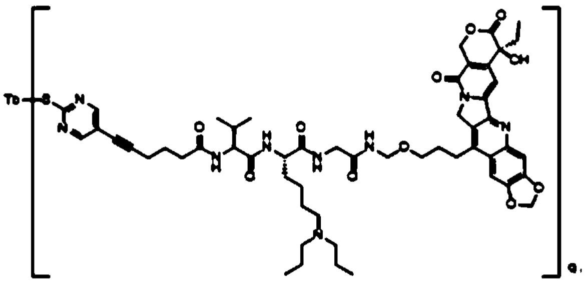

Formula I

wherein:

Tb is an anti-B7H3 antibody or an antigen-binding fragment thereof;
S is a sulfur atom on Tb;
q is any numerical value between 1 and 16, including both end values;
the nasopharyngeal carcinoma and the lymphoepithelioma-like carcinoma are as described above.

In some embodiments, in the method of treating nasopharyngeal carcinoma and/or lymphoepithelioma-like carcinoma, the anti-B7H3 antibody-drug conjugate is as described in any of the embodiments herein.
In some embodiments, in the method of treating nasopharyngeal carcinoma and/or lymphoepithelioma-like carcinoma, the definitions of the nasopharyngeal carcinoma and the lymphoepithelioma-like carcinoma, the dosage of administration, the routes of administration, the dosing cycle, and the dosing frequency are as described in any of the embodiments herein.

Single Active Ingredient

A use of an anti-B7H3 antibody-drug conjugate (preferably, formula II), a pharmaceutically acceptable salt, stereoisomer, or metabolite, or a solvate of any of the aforementioned items as a sole active ingredient in the preparation of a drug for treating nasopharyngeal carcinoma and/or lymphoepithelioma-like carcinoma, or a drug for administration alone to a patient with nasopharyngeal carcinoma.
In the above use and treatment method, the anti-B7H3 antibody-drug conjugate (preferably, formula II), a pharmaceutically acceptable salt, stereoisomer, or metabolite thereof, or a solvate of any of the aforementioned items is

used as the sole active ingredient or is administered alone to a patient with nasopharyngeal carcinoma.

Pharmaceutical Kit

The present invention provides a pharmaceutical kit, comprising (a) at least one unit dose of an anti-B7H3 antibody-drug conjugate (preferably, formula II), or a pharmaceutically acceptable salt, stereoisomer, or metabolite thereof, or a solvate of any of the aforementioned items, and (b) instructions for use in the treatment of nasopharyngeal carcinoma and/or lymphoepithelioma-like carcinoma.

Beneficial effects

Clinical trials have found that after administration of the anti-B7H3 antibody-drug conjugate of the present invention to patients with nasopharyngeal carcinoma and lymphoepithelioma-like carcinoma, the objective response rate (ORR) and the disease control rate (DCR) are significantly improved, effectively controlling the progression of the patients' tumor, exhibiting a marked inhibitory effect on tumor lesions, possessing good clinical therapeutic significance, and producing unexpected technical effects.

Detailed description of the invention

Unless otherwise defined, all terms used herein have the same meaning as commonly understood by those of ordinary skill in the art. For relevant definitions and terms, reference may be made to, for example, Current Protocols in Molecular Biology (Ausubel). At the same time, for a better understanding of the present disclosure, definitions and explanations of relevant terms are provided below.

All references mentioned herein are incorporated herein by reference in their entirety.

The B7H3 antibodies of natural sequences mentioned herein can be isolated from nature or can be prepared by recombinant DNA technology, chemical synthesis, or a combination thereof.

As used herein, the term "antibody" is used in its broadest sense and includes intact monoclonal antibodies, polyclonal antibodies, and multispecific antibodies (such as bispecific antibodies) formed from at least two intact antibodies, provided that they possess the desired biological activity.

As used herein, the term "monoclonal antibody" refers to an antibody derived from a population of essentially homogeneous antibodies, that is, each antibody constituting the population is identical, except for the possible presence of a small number of natural mutations. Monoclonal antibodies have high specificity directed against one determinant (epitope) of an antigen, whereas polyclonal antibodies in contrast contain different antibodies directed against different determinants (epitopes). In addition to specificity, monoclonal antibodies have the advantage that they can be synthesized without contamination from other antibodies. The modifier "monoclonal" here represents that the characteristic of the antibody is derived from a substantially homogeneous population of antibodies, and should not be construed as being prepared by a particular method.

Herein, monoclonal antibodies also specifically include chimeric antibodies, that is, a portion of the heavy chain and/or light chain is identical or homologous to a certain type, class or subclass of antibody, and the remaining portions are identical or homologous to a different type, class or subclass of antibody, provided that they possess the desired biological activity (see, e.g., US4,816,567; and Morrison et al., 1984, PNAS, 81: 6851-6855). Chimeric antibodies useful in the present invention include primatized antibodies, which comprise variable region antigen-binding sequences derived from non-human primates (for example, ancient monkeys, chimpanzees, and the like) and human constant region sequences.

The term "antibody fragment" refers to a portion of an antibody, preferably an antigen-binding region (for example, an antigen-binding fragment) or a variable region. Examples of antibody fragments include Fab, Fab', F(ab')2 and Fv fragments; diabodies; linear antibodies; and single-chain antibody molecules.

Herein, the term "drug" refers to a substance that inhibits or interferes with the function of a cell and/or causes cell death or damage.

The antibody-drug conjugate of the present invention may be in the form of a pharmaceutically acceptable salt, or in the form of a stereoisomer, or in the form of a metabolite, or in the form of a solvate, and the salt, the stereoisomer, or the metabolite may also be in the form of a solvate.

The term "pharmaceutically acceptable salt" refers to salts that retain the bioavailability and properties of the compound, and they are biologically or otherwise suitable for use as drugs. In many cases, the antibody-drug conjugate of the present invention may form acid addition salts and/or base addition salts by virtue of amino and/or carboxyl or similar groups present therein.

Pharmaceutically acceptable acid addition salts may be salts formed with inorganic acids or organic acids. The inorganic acids include, for example, hydrochloric acid, hydrobromic acid, sulfuric acid, nitric acid, and phosphoric acid, etc. The organic acids include, for example, acetic acid, propionic acid, glycolic acid, pyruvic acid, oxalic acid, maleic acid,

malonic acid, succinic acid, fumaric acid, tartaric acid, citric acid, benzoic acid, cinnamic acid, mandelic acid, methanesulfonic acid, ethanesulfonic acid, p-toluenesulfonic acid, and salicylic acid, etc.

Pharmaceutically acceptable base addition salts may be salts formed with inorganic bases or organic bases. The salts formed with inorganic base include, for example, sodium salt, potassium salt, lithium salt, ammonium salt, calcium salt, magnesium salt, iron salt, zinc salt, copper salt, manganese salt, and aluminum salt, particularly preferably ammonium salt, potassium salt, sodium salt, calcium salt, and magnesium salt. The organic bases include, for example, primary amines, secondary amines, and tertiary amines, substituted amines (including naturally occurring substituted amines), cyclic amines, basic ion exchange resins, etc. Specific examples of organic bases are isopropylamine, trimethylamine, diethylamine, N-ethylethanamine, tripropylamine, and ethanolamine.

Pharmaceutically acceptable salts may be obtained using standard procedures well known in the art, for example, by reacting a sufficient amount of a basic compound with a suitable acid that provides pharmaceutically acceptable anions.

The term "stereoisomer" represents an isomer formed due to at least one asymmetric center. In compounds having one or more asymmetric centers, they can produce racemes, racemic mixtures, single enantiomers, diastereomeric mixtures, and individual diastereomers. Specific individual molecules may also exist as geometric isomers (cis/trans). Unless otherwise specified, when the stereochemistry of the disclosed compound is not explicitly indicated in the name or structure thereof and it has one or more asymmetric centers, it should be understood to represent all possible stereoisomers of the described compound.

The term "solvate" represents a solvate formed by association of one or more solvent molecules with any antibody-drug conjugate of formula (I) or a pharmaceutically acceptable salt or isomer thereof. The term solvate includes hydrates (for example, hemihydrate, monohydrate, dihydrate, trihydrate, tetrahydrate, and similar hydrates).

Also included within the scope of the present disclosure are metabolites of the compounds of the present invention, that is, substances formed in vivo when the compounds of the present invention are administered. Such products may be produced by, for example, oxidation, reduction, hydrolysis, amidation, deamidation, esterification, deesterification, enzymatic cleavage, and the like of the administered compound. Accordingly, the present invention includes metabolites of the compounds of the present invention, including compounds prepared by a method of bringing a compound of the present invention in contact with a mammal for a time sufficient to produce its metabolic products.

Herein, the term "treatment" refers to a method implemented to obtain a beneficial or desired clinical result. For purposes of the present invention, beneficial or desired clinical results include, but are not limited to, alleviation of symptoms, diminishment of the extent of disease, stabilization (i.e., no longer worsening) of the state of disease, delay or slowing of development of disease, amelioration or palliation of the state of disease, and alleviation of symptoms (whether in part or in whole), whether detectable or undetectable. Furthermore, "treatment" may also refer to prolonging of survival time compared to expected survival time (if untreated).

Herein, the term "progression of disease" or "disease progression" refers to an increase of the sum of diameters of target lesions of at least 20%, taking as reference the minimum value of the sum of diameters of all target lesions throughout the study (this includes the sum of baselines if the sum of baselines is the minimum value in the study), and an absolute increase in the sum of diameters of at least 5 mm; or refers to the appearance of one or more new lesions.

Herein, the term "therapeutically effective amount" or "effective amount" refers to an amount of the anti-B7H3 antibody-drug conjugate that, when administered to a cell, tissue, or subject being treated, effectively prevents or alleviates the disease or condition to be treated. A therapeutically effective dose further refers to an amount of the antibody-drug conjugate (ADC) and/or antibody or fragment thereof sufficient to result in alleviation of symptoms, the alleviation of symptoms is, for example, treatment, cure, or amelioration of the relevant medical condition, or improvement in the treatment rate, cure rate, or mitigation rate of the condition. The effective amount for a particular subject being treated may vary depending on various factors, for example, the disease to be treated, the overall health status of the patient, the method and route as well as dose of administration, and the severity of side effects. An effective amount may be the maximum dose or dosing regimen that avoids significant side effects or toxic effects. A therapeutically effective amount will alleviate symptoms typically by at least 10%; typically by at least 20%; by at least approximately 30%; by at least 40% or at least 50%.

As used herein, the term "subject" or "patient" refers to a mammal (not limited to mammal) that is the target of treatment, observation, or experimentation, including (but not limited) to humans.

As used herein, the terms "complete response (CR)", "partial response (PR)", "stable disease (SD)", "progressive disease (PD)", "objective response rate (ORR)", "disease control rate (DCR)" are defined as follows:

When the disease is solid tumors, the therapeutic effect for the solid tumors is assessed according to the following criteria (see New response evaluation criteria in solid tumors: Revised RECIST guideline (version 1.1), E. A. Eisenhauer et al., "EUROPEAN JOURNAL OF CANCER", 45(2009), pp. 228-247) as "complete response (CR)", "partial response (PR)", "stable disease (SD)", "progressive disease (PD)", "objective response rate (ORR)", "disease control rate (DCR)". The assessment of target lesions is as follows:

Complete Response (CR): All target lesions disappear, and the short axes of all pathological lymph nodes

(including target nodes and non-target nodes) must be reduced to <10 mm, with no new lesions.

Partial Response (PR): The sum of the diameters of target lesions (using short axes for lymph nodes) is reduced by at least 30% from the baseline level. Non-target lesions show no obvious progression, with no new lesions.

Stable Disease (SD): The degree of reduction of target lesions does not reach PR, and the degree of increase does not reach the PD level, falling between the two; the minimum value of the sum of diameters may be used as a reference during the study.

Progressive Disease (PD): Using the minimum value of the sum of diameters of all measured target lesions throughout the entire experimental study as a reference, the sum of diameters shows at least a 20% relative increase (if the baseline measured value is the minimum, the baseline value is used as a reference); in addition, the absolute value of the sum of diameters of measured target lesions must have an increase of at least 5 mm (the appearance of one or more new lesions is also considered as a progressive disease).

Objective Response Rate (ORR): The percentage of patients with tumor shrinkage reaching a certain degree, including cases of CR and PR. Subjects must have measurable tumor lesions at baseline, and the therapeutic effect assessment criteria are based on RECIST 1.1.

Disease Control Rate (DCR): The percentage of patients who achieved complete response (CR) and partial response (PR) as well as stable disease (SD) in the total number of analyzed people.

**Brief Description of the Drawings**

FIG. 1: Show the changes in tumor response over treatment time in 7 patients with nasopharyngeal carcinoma.

FIG. 2: Show the best percent change in the sum of diameters of target lesions in 7 patients with nasopharyngeal carcinoma.

FIG. 3: Show the changes in tumor lesions of patient 201008 with nasopharyngeal carcinoma before and after treatment with an anti-B7H3 antibody-drug conjugate.

FIG. 4: Show the changes in tumor lesions of patient 201007 with lymphoepithelioma-like carcinoma before and after treatment with the anti-B7H3 antibody-drug conjugate.

**Detailed Description of Embodiments**

The following description of specific embodiments provides further illustration of the present disclosure, but this is not a limitation of the present disclosure. Those skilled in the art may make various modifications or improvements based on the teachings of the present disclosure without departing from the basic concepts and scope of the present disclosure.

**Sequences and their specific information:**

Table 1

| SEQ ID NO: | Name | Amino acid sequence |
|---|---|---|
| 1 | 2E3/2E3-02 CDR-H1 | SYAMN |
| 2 | 2E3/2E3-02 CDR-H2 | GISGSGGSTYYADSVQG |
| 3 | 2E3/2E3-02 CDR-H3 | ARSGYDYFFDY |
| 4 | 2E3/2E3-02 CDR-L1 | RASQRISSSFLA |
| 5 | 2E3/2E3-02 CDR-L2 | GASSRAT |
| 6 | 2E3/2E3-02 CDR-L3 | QQYSNSPLT |
| 7 | 2E3/2E3-02 VH | EVQLLESGGGLVQPGGSLRLSCAASGFTFSSYAMN WVRQAPGKGLEWVSGISGSGGSTYYADSVQGRFTI SRDNSKNTLYLQMNSLRAEDTAVYYCAKARSGYD YFFDYWGQGTLVTVSS |

(continued)

| SEQ ID NO: | Name | Amino acid sequence |
|---|---|---|
| 8 | 2E3/2E3-02 VL | EIVLTQSPGTLSLSPGERATLSCRASQRISSSFLAWY<br>QQKPGQAPSLLIYGASSRATGIPDRFSGSGSGTDFT<br>LTISSLEPEDFAVFYCQQYSNSPLTFGGGTKVEIK |
| 9 | 2E3-02 HC | EVQLLESGGGLVQPGGSLRLSCAASGFTFSSYAMN<br>WVRQAPGKGLEWVSGISGSGGSTYYADSVQGRFTI<br>SRDNSKNTLYLQMNSLRAEDTAVYYCAKARSGYD<br>YFFDYWGQGTLVTVSSASTKGPSVFPLAPSSKSTSG<br>GTAALGCLVKDYFPEPVTVSWNSGALTSGVHTFPA<br>VLQSSGLYSLSSVVTVPSSSLGTQTYICNVNHKPSN<br>TKVDKKVEPKSCDKTHTCPPCPAPELLGGPSVFLFP<br>PKPKDTLMISRTPEVTCVVVDVSHEDPEVKFNWYV<br>DGVEVHNAKTKPREEQYNSTYRVVSVLTVLHQD<br>WLNGKEYKCKVSNKALPAPIEKTISKAKGQPREPQ<br>VYTLPPSRDELTKNQVSLTCLVKGFYPSDIAVEWE<br>SNGQPENNYKTTPPVLDSDGSFFLYSKLTVDKSRW<br>QQGNVFSCSVMHEALHNHYTQKSLSLSPGK |
| 10 | 2E3-02 LC | EIVLTQSPGTLSLSPGERATLSCRASQRISSSFLAWY<br>QQKPGQAPSLLIYGASSRATGIPDRFSGSGSGTDFT<br>LTISSLEPEDFAVFYCQQYSNSPLTFGGGTKVEIKRT<br>VAAPSVFIFPPSDEQLKSGTASVVCLLNNFYPREAK<br>VQWKVDNALQSGNSQESVTEQDSKDSTYSLSSTLT<br>LSKADYEKHKVYACEVTHQGLSSPVTKSFNRGEC |

Embodiment 1: Production of anti-B7H3 antibody-drug conjugate

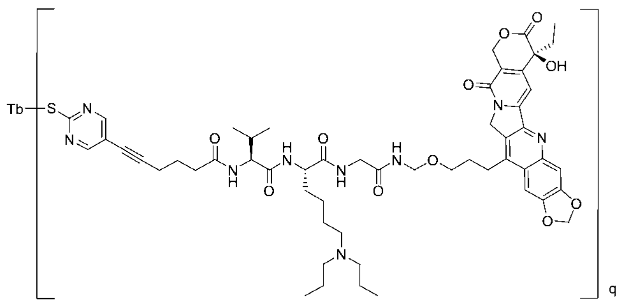

Formula I

With reference to the production method described in Patent Application WO2022170971A1, a humanized B7H3 antibody was used to produce the anti-B7H3 antibody-drug conjugate as shown in Formula I.

Embodiment 2: Production of anti-B7H3 antibody-drug conjugate (1)

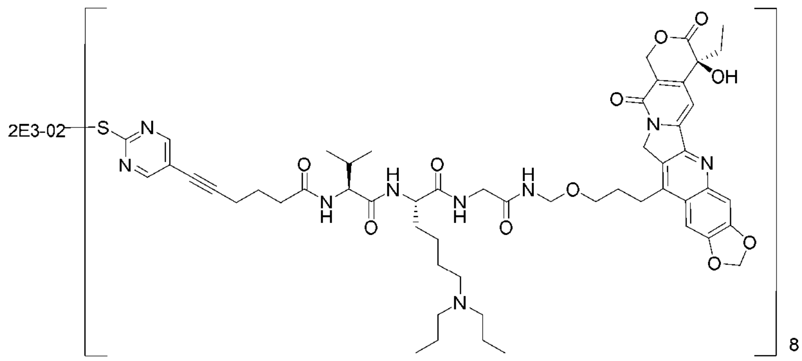

With reference to the production method described in Patent Application WO2023143208A1, a humanized B7H3 antibody (2E3-02) was used to produce the anti-B7H3 antibody-drug conjugate as shown above (hereinafter referred to as "anti-B7H3 antibody-drug conjugate (1)").

Embodiment 3: Clinical trial of anti-B7H3 antibody-drug conjugate in the treatment of nasopharyngeal carcinoma

This trial is a phase I, multicenter, non-randomized, open-label, first-in-human study to evaluate the safety, tolerance, pharmacokinetics, and efficacy of the anti-B7H3 antibody-drug conjugate of the present invention in patients with nasopharyngeal carcinoma.

1. The selection criteria for subjects are as follows:

The inclusion criteria are as follows:

1) having been informed of the trial situation before the start of the trial, and voluntarily signing the names and dates on the informed consent forms;
2)

at age ≥18 years;

3) the Eastern Cooperative Oncology Group Performance Status (ECOG PS) score is 0 or 1;
4) organ and bone marrow functions meet requirements, which are defined as follows:

- hemoglobin ≥90 g/L (no blood transfusion or erythropoietin treatment was received within 14 days prior to the first dosing);
- absolute neutrophil count $\geq 1.5 \times 10^9$/L (no granulocyte colony-stimulating factor or granulocyte-macrophage colony-stimulating factor treatment was received within 14 days prior to first dosing);
- platelet count $\geq 100 \times 10^9$/L in the absence of apparent hepatic lesions (primary or metastatic), or $\geq 75 \times 10^9$/L in the presence of hepatic lesions (no platelet transfusion, thrombopoietin, or interleukin-11 treatment was received within 14 days prior to the first dosing);
- total bilirubin ≤1.5 × ULN in the absence of apparent hepatic lesions (primary or metastatic), or ≤3 × ULN in the presence of hepatic lesions;
- ALTand AST ≤3 × ULN in the absence of apparent hepatic lesions (primary or metastatic), or ≤5 × ULN in the presence of hepatic lesions;
- creatinine clearance ≥60 mL/min (calculated according to the Cockcroft-Gault formula);
- activated partial thromboplastin time and international normalized ratio ≤1.5 × ULN in patients not receiving anticoagulant treatment, or stable anticoagulation regimens in patients receiving anticoagulant treatment;

5) female patients having ability to have children must agree to use highly effective contraception from the time of screening, throughout the entire study period, and for at least 6 months after the last dosing of study drugs, and must not donate oocytes or retrieve oocytes for personal use. Male patients must agree to use highly effective contraception from the time of screening, throughout the entire study period, and for at least 6 months after the last dosing of study drugs, and must not cryopreserve or donate sperm;
6)

expected lifetime ≥3 months;

7) being able and willing to comply with the visits and procedures required by the study protocol;
8) being confirmed by pathology as advanced solid tumors (including nasopharyngeal carcinoma), where prior standard treatment has been proven ineffective or intolerable, or no standard treatment is available;
9) there was at least 1 evaluable tumor lesion according to Response Evaluation Criteria in Solid Tumors (RECIST) version 1.1.

The exclusion criteria are as follows:

1) having previously received treatment with a B7H3-targeted drug (including antibodies, antibody-drug conjugates [ADC], chimeric antigen receptor T cells [CAR-T], and other drugs);
2) having intolerance when previously treated with topoisomerase I inhibitors or ADC composed of topoisomerase I inhibitors, including but not limited to topotecan, irinotecan, and Dxd (for example, severe diarrhea);
3) being simultaneously enrolled in another clinical study, unless it is an observational (non-interventional) clinical study or in the follow-up phase of an interventional study;
4) having received systemic anti-cancer treatment within 3 weeks prior to the first dosing of study drugs, including chemotherapy, molecular targeted treatment, hormone treatment, immunotherapy, or biotherapy (oral fluorouracil [for example, tegafur and capecitabine] or small molecule targeted treatment within 2 weeks or 5 half-lives prior to the first dosing [whichever is shorter]; mitomycin or nitrosourea drugs within 6 weeks prior to the first dosing; Chinese herbal drugs or non-specific immunomodulators [for example, thymosin, interferon, and interleukin] with anti-tumor indications within 2 weeks prior to the first dosing);
5) having received radiotherapy within 4 weeks prior to the first dosing of study drugs, including palliative stereotactic radiotherapy to the abdomen (palliative stereotactic radiotherapy to non-abdominal areas within 2 weeks prior to the first dosing);
6) having received major surgeries (excluding diagnostic surgeries) within 4 weeks prior to the first dosing of study drugs, or major surgeries anticipated during the study period;
7) having received allogeneic hematopoietic stem cell transplantation (HSCT) prior to the first dosing of study drugs, or autologous HSCT within 3 months prior to the first dosing of study drugs;
8) having received systemic corticosteroids (>10 mg/day of prednisone or equivalent) or other immunosuppressive treatment received within 2 weeks prior to the first dosing of study drugs. These criteria are not applied in the following situations:

- intranasal, inhaled, topical corticosteroids, or local corticosteroid injection (such as intra-articular injection);
- physiologic dosages of systemic corticosteroids as replacement therapies (such as physiologic corticosteroid replacement therapies against adrenal or pituitary insufficiency);
- corticosteroids as prophylactic drugs for hypersensitivity reactions (such as prophylactic drugs for CT scanning);

9) having received any live vaccine within 4 weeks prior to the first dosing of study drugs, or planned to receive live vaccine during the study period;
10) having history of leptomeningeal carcinomatosis;
11) having brain metastases or spinal cord compression, unless asymptomatic, or have stable symptoms after treatment and have no requirement of treatment with corticosteroids or anticonvulsant drugs within at least 2 weeks prior to the first dosing of study drugs;
12) having uncontrolled or clinically significant cardiovascular diseases, including but not limited to:

- history of symptomatic congestive heart failure (New York Heart Association [NYHA] class II to IV) or any arterial thromboembolism events (such as myocardial infarction, unstable angina, cerebrovascular accidents, and transient ischemic attacks) within 6 months prior to the first dosing;
- uncontrolled hypertension, defined as systolic blood pressure (SBP) >160 mmHg and/or diastolic blood pressure (DBP) >100 mmHg after antihypertensive treatment;
- severe arrhythmia requiring treatment;
- QT interval corrected by Fridericia formula (QTcF) prolonged to >470 ms;

13) history of the (non-infectious) interstitial lung disease (ILD)/pulmonary inflammation requiring corticosteroid treatment, or current ILD/pulmonary inflammation, or suspected ILD/pulmonary inflammation that cannot be excluded by imaging at screening;
14) clinically significant concomitant pulmonary diseases, including but not limited to:

- pulmonary embolism occurring within 3 months prior to initiation of study treatment;
- any recorded autoimmune, connective tissue, or inflammatory diseases at screening (for example, rheumatoid arthritis, Sjögren syndrome, sarcoidosis), or suspected pulmonary involvement;
- previous pneumonectomy;

15) diagnosed with Gilbert syndrome;
16) being accompanied with uncontrolled third-space fluid accumulation (for example, pleural effusion, ascites, pericardial effusion) and requiring repeated drainage;
17) having active gastric and duodenal ulcers, ulcerative colitis, or other gastrointestinal diseases that in the opinion of the investigator may cause bleeding or perforation;
18) having uncontrolled infection within 1 week prior to the first dosing and requiring systemic treatment;
19) having known human immunodeficiency virus (HIV) infection;
20) having active hepatitis B virus (HBV) or hepatitis C virus (HCV) infection. Active HBV is defined as being positive for hepatitis B core antibody (HBcAb) or hepatitis B surface antigen (HBsAg), with the HBV DNA level above the ULN of the study center; active HCV is defined as being positive for hepatitis C antibody, with the HCV RNA level above the ULN of the study center;
21) no recovery of toxicity from previous anti-cancer therapy is defined as toxicity not recovered to NCI CTCAE ≤ grade 1, the baseline level, or the level specified in the inclusion/exclusion criteria, with the exception of alopecia (any grade), pigmentation (any grade), and peripheral neuropathy (≤ grade 2). patients who have irreversible toxicities (such as hearing loss) and are not exacerbated by study drug according to reasonable expectations may be enrolled after discussion with the sponsor;
22) having history of severe hypersensitivity reactions to active pharmaceutical ingredients, inactive ingredients in preparations, or other monoclonal antibodies;
23) lactating women, or women confirmed to be pregnant by pregnancy testing within 3 days prior to the first dosing;
24) the presence of any disease, medical condition, organ system dysfunction, or social situation, including but not limited to mental illness or substance/alcohol abuse, in the opinion of the investigator that may interfere with the patient's ability to provide informed consent, adversely affect the patient's ability to cooperate and participate in the study, or affect the interpretation of study results;

2. Treatment regimen: subjects meeting all inclusion criteria and not meeting any exclusion criteria received the monotherapy of the anti-B7H3 antibody-drug conjugate (1) (lyophilized injection prepared by conventional methods in the art) (with a dosage of administration of 0.8, 1.6, 2.0, 2.4, 2.8, or 3.0 mg/kg, intravenous infusion, once every three weeks as one cycle), and are subjected to efficacy evaluation once every 2 dosing cycles until disease progression, intolerable toxicity, or voluntary withdrawal occurs. The study endpoint criteria include safety, ORR, DCR, pharmacokinetics, immunogenicity, and the like.
3. Therapeutic effect: A total of 7 patients with nasopharyngeal carcinoma who failed standard treatment were enrolled in the clinical study, with 5 patients achieving PR, 2 patients achieving SD, the overall ORR of 71.4% (5/7), and the DCR of 100% (7/7). See FIG. 1 and FIG. 2 for details, and specific therapeutic effects are presented in Embodiment 4.It can be seen that the anti-B7H3 antibody-drug conjugate of the present invention can effectively control tumor lesions in patients with nasopharyngeal carcinoma, and the tumor condition is gradually improved.

Embodiment 4: Detailed situations of therapeutic effects of anti-B7H3 antibody-drug conjugate on patients with nasopharyngeal carcinoma

Patient 201008: Male, 39 years old, pathologically diagnosed with nasopharyngeal carcinoma, and having multiple pulmonary metastases, and cervical lymph node metastases. After 18 weeks of treatment with the anti-B7H3 antibody-drug conjugate (1), the tumor lesion located at the inferior lobe of the right lung was reduced from 25 mm to 5 mm, with a reduction of 80%, see FIG. 3.

Patient 201012: Male, 28 years old, pathologically diagnosed with nasopharyngeal carcinoma, and having pulmonary metastases, cervical and parotid lymph node metastases, and bone metastases. After 12 weeks of treatment with the anti-B7H3 antibody-drug conjugate (1), the tumor lesion located at the cervical region was reduced from 15 mm to 5 mm, with a reduction of 66.7%.

Patient 201017: Male, 53 years old, pathologically diagnosed with nasopharyngeal carcinoma, and having multiple pulmonary metastases, and mediastinal and hilum lymph node metastases. After 6 weeks of treatment with the anti-B7H3 antibody-drug conjugate (1), the tumor lesion located at the middle lobe of the right lung was reduced from 15 mm to 9 mm, with a reduction of 40%.

Patient 201018: Male, 41 years old, pathologically diagnosed with nasopharyngeal carcinoma, and having pulmonary metastases, hepatic metastases, cerebral metastases, mediastinal and hilum lymph node metastases, and bone metastases after left superior pulmonary lobectomy + left inferior lobe nodule resection + right parieto-occipital lobe craniotomy tumor resection. After 12 weeks of treatment of the tumor lesions located at hepatic segments S7, S2 with the anti-B7H3 antibody-drug conjugate (1), the tumor lesion located at the hepatic segment S7 was reduced from 44 mm to 28 mm, with a reduction of 36.4%; the tumor lesion located at the hepatic segment S2 was reduced from 23 mm to 16 mm, with a reduction of 30.4%.

Patient 201019: Male, 56 years old, pathologically diagnosed with nasopharyngeal carcinoma, and having multiple pulmonary metastases, hepatic metastases, renal metastases, pleural metastases, pulmonary hilum and mediastinal lymph node metastases, and bone metastases; after 12 weeks of treatment of tumor lesions located at the middle lobe of the right lung and the superior lobe of the left lung with the anti-B7H3 antibody-drug conjugate (1), the tumor lesion at the middle lobe of the right lung was reduced from 13 mm to 7 mm, with a reduction of 46.2%; the tumor lesion at the superior lobe of the left lung was reduced from 13 mm to 9 mm, with a reduction of 30.8%.

Embodiment 5: Clinical trial of anti-B7H3 antibody-drug conjugate in the treatment of lymphoepithelioma-like carcinoma

This trial is a phase I, multicenter, non-randomized, open-label, first-in-human study to evaluate the safety, tolerance, pharmacokinetics, and efficacy of the anti-B7H3 antibody-drug conjugate of the present invention in patients with lymphoepithelioma-like carcinoma.

1. The selection criteria for subjects are as follows:

The inclusion criteria are as follows:

1) having been informed of the trial situation before the start of the trial, and voluntarily signing the names and dates on the informed consent forms;
2)

at age ≥18 years;

3) the Eastern Cooperative Oncology Group Performance Status (ECOG PS) score is 0 or 1;
4) organ and bone marrow functions meet requirements, which are defined as follows:

- hemoglobin ≥90 g/L (no blood transfusion or erythropoietin treatment was received within 14 days prior to the first dosing);
- absolute neutrophil count $\geq 1.5 \times 10^9$/L (no granulocyte colony-stimulating factor or granulocyte-macrophage colony-stimulating factor treatment was received within 14 days prior to first dosing);
- platelet count $\geq 100 \times 10^9$/L in the absence of apparent hepatic lesions (primary or metastatic), or $\geq 75 \times 10^9$/L in the presence of hepatic lesions (no platelet transfusion, thrombopoietin, or interleukin-11 treatment was received within 14 days prior to the first dosing);
- total bilirubin ≤1.5 × ULN in the absence of apparent hepatic lesions (primary or metastatic), or ≤3 × ULN in the presence of hepatic lesions;
- ALTand AST ≤3 × ULN in the absence of apparent hepatic lesions (primary or metastatic), or ≤5 × ULN in the presence of hepatic lesions;
- creatinine clearance ≥60 mL/min (calculated according to the Cockcroft-Gault formula);
- activated partial thromboplastin time and international normalized ratio ≤1.5 × ULN in patients not receiving anticoagulant treatment, or stable anticoagulation regimens in patients receiving anticoagulant treatment;

5) female patients having ability to have children must agree to use highly effective contraception from the time of screening, throughout the entire study period, and for at least 6 months after the last dosing of study drugs, and must not donate oocytes or retrieve oocytes for personal use. Male patients must agree to use

highly effective contraception from the time of screening, throughout the entire study period, and for at least 6 months after the last dosing of study drugs, and must not cryopreserve or donate sperm;
6)

expected lifetime ≥3 months;

7) being able and willing to comply with the visits and procedures required by the study protocol;
8) being confirmed by pathology as advanced solid tumors, where prior standard treatment has been proven ineffective or intolerable, or no standard treatment is available;
9) there was at least 1 evaluable tumor lesion according to Response Evaluation Criteria in Solid Tumors (RECIST) version 1.1.

The exclusion criteria are as follows:

1) having previously received treatment with a B7H3-targeted drug (including antibodies, antibody-drug conjugates [ADC], chimeric antigen receptor T cells [CAR-T], and other drugs);
2) having intolerance when previously treated with topoisomerase I inhibitors or ADC composed of topoisomerase I inhibitors, including but not limited to topotecan, irinotecan, and Dxd (for example, severe diarrhea);
3) being simultaneously enrolled in another clinical study, unless it is an observational (non-interventional) clinical study or in the follow-up phase of an interventional study;
4) having received systemic anti-cancer treatment within 3 weeks prior to the first dosing of study drugs, including chemotherapy, molecular targeted treatment, hormone treatment, immunotherapy, or biotherapy (oral fluorouracil [for example, tegafur and capecitabine] or small molecule targeted treatment within 2 weeks or 5 half-lives prior to the first dosing [whichever is shorter]; mitomycin or nitrosourea drugs within 6 weeks prior to the first dosing; Chinese herbal drugs or non-specific immunomodulators [for example, thymosin, interferon, and interleukin] with anti-tumor indications within 2 weeks prior to the first dosing);
5) having received radiotherapy within 4 weeks prior to first dosing of study drugs, including palliative stereotactic radiotherapy to the abdomen (palliative stereotactic radiotherapy to non-abdominal areas within 2 weeks prior to the first dosing);
6) having received major surgeries (excluding diagnostic surgeries) within 4 weeks prior to the first dosing of study drugs, or major surgeries anticipated during the study period;
7) having received allogeneic hematopoietic stem cell transplantation (HSCT) prior to the first dosing of study drugs, or autologous HSCT within 3 months prior to the first dosing of study drugs;
8) having received systemic corticosteroids (>10 mg/day of prednisone or equivalent) or other immunosuppressive treatment received within 2 weeks prior to the first dosing of study drugs. These criteria are not applied in the following situations:

- intranasal, inhaled, topical corticosteroids, or local corticosteroid injection (such as intra-articular injection);
- physiologic dosages of systemic corticosteroids as replacement therapies (such as physiologic corticosteroid replacement therapies against adrenal or pituitary insufficiency);
- corticosteroids as prophylactic drugs for hypersensitivity reactions (such as prophylactic drugs for CT scanning);

9) having received any live vaccine within 4 weeks prior to the first dosing of study drugs, or planned to receive live vaccine during the study period;
10) having history of leptomeningeal carcinomatosis;
11) having brain metastases or spinal cord compression, unless asymptomatic, or have stable symptoms after treatment and have no requirement of treatment with corticosteroids or anticonvulsant drugs within at least 2 weeks prior to the first dosing of study drugs;
12) having uncontrolled or clinically significant cardiovascular diseases, including but not limited to:

- history of symptomatic congestive heart failure (New York Heart Association [NYHA] class II to IV) or any arterial thromboembolism events (such as myocardial infarction, unstable angina, cerebrovascular accidents, and transient ischemic attacks) within 6 months prior to the first dosing;
- uncontrolled hypertension, defined as systolic blood pressure (SBP) >160 mmHg and/or diastolic blood

pressure (DBP) >100 mmHg after antihypertensive treatment;

- severe arrhythmia requiring treatment;
- QT interval corrected by Fridericia formula (QTcF) prolonged to >470 ms;

13) history of the (non-infectious) interstitial lung disease (ILD)/pulmonary inflammation requiring corticosteroid treatment, or current ILD/pulmonary inflammation, or suspected ILD/pulmonary inflammation that cannot be excluded by imaging at screening;
14) clinically significant concomitant pulmonary diseases, including but not limited to:

- pulmonary embolism occurring within 3 months prior to initiation of study treatment;
- any recorded autoimmune, connective tissue, or inflammatory diseases at screening (for example, rheumatoid arthritis, Sjögren syndrome, sarcoidosis), or suspected pulmonary involvement;
- previous pneumonectomy;

15) diagnosed with Gilbert syndrome;
16) being accompanied with uncontrolled third-space fluid accumulation (for example, pleural effusion, ascites, pericardial effusion) and requiring repeated drainage;
17) having active gastric and duodenal ulcers, ulcerative colitis, or other gastrointestinal diseases that in the opinion of the investigator may cause bleeding or perforation;
18) having uncontrolled infection within 1 week prior to the first dosing and requiring systemic treatment;
19) having known human immunodeficiency virus (HIV) infection;
20) having active hepatitis B virus (HBV) or hepatitis C virus (HCV) infection. Active HBV is defined as being positive for hepatitis B core antibody (HBcAb) or hepatitis B surface antigen (HBsAg), with the HBV DNA level above the ULN of the study center; active HCV is defined as being positive for hepatitis C antibody, with the HCV RNA level above the ULN of the study center;
21) no recovery of toxicity from previous anti-cancer therapy is defined as toxicity not recovered to NCI CTCAE ≤ grade 1, the baseline level, or the level specified in the inclusion/exclusion criteria, with the exception of alopecia (any grade), pigmentation (any grade), and peripheral neuropathy (≤ grade 2). patients who have irreversible toxicities (such as hearing loss) and are not exacerbated by study drug according to reasonable expectations may be enrolled after discussion with the sponsor;
22) having history of severe hypersensitivity reactions to active pharmaceutical ingredients, inactive ingredients in preparations, or other monoclonal antibodies;
23) lactating women, or women confirmed to be pregnant by pregnancy testing within 3 days prior to the first dosing;
24) the presence of any disease, medical condition, organ system dysfunction, or social situation, including but not limited to mental illness or substance/alcohol abuse, in the opinion of the investigator that may interfere with the patient's ability to provide informed consent, adversely affect the patient's ability to cooperate and participate in the study, or affect the interpretation of study results;

2. Treatment regimen: subjects meeting all inclusion criteria and not meeting any exclusion criteria received the monotherapy of the anti-B7H3 antibody-drug conjugate (1) (lyophilized injection prepared by conventional methods in the art) (with a dosage of administration of 0.8, 1.6, 2.0, 2.4, 2.8, or 3.0 mg/kg, intravenous infusion, once every three weeks as one cycle), and are subjected to efficacy evaluation once every 2 dosing cycles until disease progression, intolerable toxicity, or voluntary withdrawal occurs.The study endpoint criteria include safety, ORR, DCR, pharmacokinetics, immunogenicity, and the like.
3. Therapeutic effect: A total of 4 patients with lymphoepithelioma-like carcinoma who failed standard treatment were enrolled in the clinical study, with 3 patients achieving PR, 1 patient achieving SD, the overall ORR of 75% (3/4), and the DCR of 100% (4/4), and detailed situations of therapeutic effects were presented in Embodiment 6.It can be seen that the anti-B7H3 antibody-drug conjugate of the present invention can effectively control tumor lesions in patients with lymphoepithelioma-like carcinoma, and the tumor condition is gradually improved.

Embodiment 6: Detailed conditions of therapeutic effects of anti-B7H3 antibody-drug conjugate on patients with lymphoepithelioma-like carcinoma

Patient 201007: Female, 64 years old, pathologically diagnosed with lymphoepithelioma-like carcinoma, and having multiple pulmonary metastases, hepatic metastases, and cervical, thoracic, and abdominal lymph node metastases. After 24 weeks of treatment of tumor lesions located at the middle lobe of the right lung, cervical region, and hepatic segment S7 with the anti-B7H3 antibody-drug conjugate (1), the tumor lesions located at the middle lobe of the right lung

were reduced from 23 mm to 19 mm, with a reduction of 17.4%; the tumor lesions at the cervical region were reduced from 19 mm to 12 mm, with a reduction of 36.8%; the tumor lesions at the hepatic segment S7 were reduced from 16 mm to 8 mm, with a reduction of 50%, as shown in FIG. 4.

Patient 201016: Female, 60 years old, pathologically diagnosed with lymphoepithelioma-like carcinoma, and having multiple pulmonary metastases, hepatic metastases, subpleural metastases, gluteal metastases, cervical, thoracic, and abdominal lymph node metastases, and bone metastases. After 6 weeks of treatment of tumor lesions located at the cervical region and subpleural region with the anti-B7H3 antibody-drug conjugate (1), the tumor lesions located at the cervical region were reduced from 15 mm to 6 mm, with a reduction of 60%; the tumor lesions located at the subpleural region were reduced from 16 mm to 15 mm, with a reduction of 6.3%.

Patient 201025: Female, 46 years old, pathologically diagnosed with lymphoepithelioma-like carcinoma, and having multiple pulmonary metastases, lung hilar metastases, pleural metastases, and mediastinal, lung hilar, and clavicular lymph node metastases; after 6 weeks of treatment of tumor lesions located at the inferior lobe of the left lung, superior lobe of the left lung, and clavicle with the anti-B7H3 antibody-drug conjugate (1), the tumor lesions located at the inferior lobe of the left lung were reduced from 11 mm to 9 mm, with a reduction of 18.2%; the tumor lesions at the superior lobe of the left lung were reduced from 13 mm to 7 mm, with a reduction of 46.2%; the tumor lesions located at the clavicle were reduced from 15 mm to 11 mm, with a reduction of 26.7%.

Embodiment 7: Clinical trial of anti-B7H3 antibody-drug conjugate in the treatment of selected advanced solid tumors

This study is a multicenter, open-label, phase I/II study of the anti-B7H3 antibody-drug conjugate for evaluating the safety, efficacy, and PK characteristics of the anti-B7H3 antibody-drug conjugate in patients with selected advanced solid tumors:

**I. Inclusion Criteria**

Subjects must meet all of the following criteria to be enrolled in the study:

1) Having been informed of the trial situation before the start of the trial, and voluntarily signing the names and dates on the informed consent forms (ICF).
2) At age ≥18 years and ≤75 years when signing the ICF.
3) Subjects enrolled from the NPC Cohort must meet the following conditions:

- being histologically or cytologically diagnosed with NPC;
- having metastatic (Stage IVB, using UICC and AJCC Staging Systems, 8th edition) or recurrent diseases that are not suitable for local or definitive treatment at the time of enrollment;
- for metastatic or recurrent diseases, having previously received at least platinum-containing chemotherapy and anti-PD-(L)1 treatment, and having recorded disease progression or intolerance during or after treatment.

Subjects enrolled from the Other Solid Tumor Cohorts must meet the following conditions:

- being histologically or cytologically diagnosed with advanced solid tumor;
- having recorded disease progression or intolerance during or after previously receiving standard treatment, or having no standard treatment available. **Note:** For all cohorts described above, if a subject has disease progression during or within 6 months after previously receiving neoadjuvant therapy, adjuvant therapy, or definitive chemoradiation therapy, such treatment regimen will be considered as first-line treatment against advanced/metastatic diseases.

4) There is at least 1 extracranial measurable lesion according to RECIST v1.1. Lesions previously receiving radiotherapy or other local treatment cannot be used as target lesions, unless the lesions show clear progression. For mCRPC patients with only bone lesions, enrollment may be determined on a case-by-case basis after discussion with the sponsor.
5) Archived or fresh tumor tissue samples can be provided. For subjects unable to provide tumor samples or with insufficient samples, enrollment may be determined on a case-by-case basis after discussion with the sponsor.
6) The Eastern Cooperative Oncology Group Performance Status (ECOG PS) score is 0 or 1.
7) Organ and bone marrow functions meet requirements within 7 days before the first dosing, which are defined as follows:

- hemoglobin (Hb) ≥9.0 g/dL (no blood transfusion or erythropoietin treatment were received within 14 days prior to

first dosing);

- absolute neutrophil count (ANC) 1.5×$10^9$/L (no granulocyte colony-stimulating factor or granulocyte-macrophage colony-stimulating factor treatment was received within 14 days prior to the first dosing);
- platelet count (PLT) ≥100×$10^9$/L (no platelet transfusion, thrombopoietin, or interleukin-11 treatment was received within 14 days prior to the first dosing);
- total bilirubin (TBIL) ≤1.5× upper limit of normal value (ULN) in the absence of significant hepatic metastases, or ≤3×ULN in the presence of hepatic metastases;
- alanine aminotransferase (ALT) and aspartate aminotransferase (AST) ≤3×ULN in the absence of significant hepatic metastases, or ≤5×ULN in the presence of hepatic metastases;
- serum albumin ≥2.5 g/dL;
- creatinine clearance ≥50 mL/min (calculated according to the Cockcroft-Gault formula);
- activated partial thromboplastin time (APTT) and international normalized ratio (INR) ≤1.5×ULN, except for subjects receiving anticoagulation therapy, where these subjects must have a stable anticoagulation regimen, and APTT and INR must be within the therapeutic range in the opinion of the investigator.

8) Female subjects having ability to have children must agree to use highly effective contraception from the time of screening, throughout the entire study period, and for at least 6 months after the last dosing of trial drugs. Male subjects must agree to use highly effective contraception from the time of screening, throughout the entire study period, and for at least 6 months after the last dosing of trial drugs.
9)

Expected lifetime ≥3 months.

10) Be able and willing to comply with the visits and procedures required by the study protocol.

**II. Exclusion Criteria**

Subjects meeting any of the following criteria shall not be enrolled in the study:

1) having previously received treatment with a B7H3-targeted drug (including antibodies, antibody-drug conjugates [ADC], chimeric antigen receptor T cells [CAR-T], and other drugs);
2) having previously received a topoisomerase I inhibitor or ADC consisting of a topoisomerase I inhibitor;
3) being simultaneously enrolled in another clinical study, unless it is an observational (non-interventional) clinical study or in the follow-up phase of an interventional study.
4) The washout period of the previous anti-cancer treatment before the first dosing of the trial drugs is inadequate, defined as follows:

- chemotherapy or small molecule targeted therapy <2 weeks or 5 half-lives, whichever is shorter;
- antibody treatment <3 weeks;
- hormonal treatment <3 weeks;
- herbal or botanical treatment with anti-tumor indications <2 weeks;
- brain radiotherapy <2 weeks;
-

palliative radiotherapy <2 weeks; definitive radiotherapy <4 weeks.

5) Having received major surgeries (excluding diagnostic surgeries) within 4 weeks prior to the first dosing of trial drugs, or major surgeries anticipated during the study period.
6) Having previously received allogeneic bone marrow transplantation or previously received solid organ transplantation.
7) Having received systemic corticosteroids (>10 mg/day of prednisone or equivalent) or other immunosuppressive treatment received within 2 weeks prior to the first dosing of trial drugs, with the following exceptions:

- intranasal, inhaled, topical corticosteroids, or local corticosteroid injection (such as intra-articular injection);
- physiologic dosages of systemic corticosteroids as replacement therapies (such as physiologic corticosteroid replacement therapies against adrenal or pituitary insufficiency);
- corticosteroids are used as prophylactic drugs for hypersensitivity reactions (such as CT contrast allergy) or chemotherapy-induced nausea and vomiting (CINV).

8) Any live vaccine has been received within 4 weeks prior to the first dosing of the trial drugs, or live vaccine is planned to be received during the study period.
9) Leptomeningeal metastases or carcinomatous meningitis.
10) Brain metastases or spinal cord compression, with the following exceptions:

- subjects with asymptomatic brain metastases not requiring immediate local or systemic treatment (such as mannitol or corticosteroids) are permitted to be enrolled;
- subjects shall be allowed to be enrolled if their brain metastases have been treated and the metastases are stable in state (brain imaging examination at least 4 weeks before the first dosing shows stable lesions, there are no new neurological symptoms, and no immediate local or systemic treatment is required within 2 weeks before the first dosing), and there is no evidence of new or enlarged original brain metastases.

11) Having uncontrolled or clinically significant cardiovascular diseases, including but not limited to:

- history of symptomatic congestive heart failure (New York Heart Association [NYHA] Class II to IV) or any arterial thromboembolic event (such as myocardial infarction, unstable angina, cerebrovascular accident, and transient ischemic attack) within 6 months prior to the first dosing.
- uncontrolled hypertension, defined as systolic blood pressure (SBP) >160 mmHg and/or diastolic blood pressure (DBP) >100 mmHg after antihypertensive treatment;
- severe arrhythmia requiring treatment;
- QT interval corrected by Fridericia formula (QTcF) prolonged to >450 ms (male) or 470 ms (female).

12) clinically significant concomitant pulmonary diseases, including but not limited to:

- history of (non-infectious) interstitial lung disease (ILD)/interstitial pneumonitis requiring corticosteroid treatment, or current ILD/interstitial pneumonitis;
- having pulmonary embolism within 3 months prior to the first dosing;
- having any recorded autoimmune, connective tissue, or inflammatory disease at screening (such as rheumatoid arthritis, Sjögren syndrome, sarcoidosis), or suspected pulmonary involvement;
- previous pneumonectomy;
- other moderate to severe pulmonary diseases seriously affecting respiratory functions that may interfere with the detection or handling of drug-related pulmonary toxicity.

13) Being diagnosed as Gilbert syndrome.
14) Being accompanied with uncontrolled third-space fluid accumulation (such as pleural effusion, ascites, or pericardial effusion) requiring repeated drainage.
15) Having gastrointestinal perforation and/or fistula within 6 months prior to the first dosing, or having active gastric and duodenal ulcer, ulcerative colitis, or other gastrointestinal diseases that in the opinion of the investigator may cause bleeding or perforation.
16) Serious infection (National Cancer Institute Common Terminology Criteria for Adverse Events [NCI CTCAE] ≥3 grade) within 4 weeks prior to the first dosing, such as serious pneumonia, bacteremia, infection complications, etc. requiring hospitalization; or active infection requiring systemic treatment within 2 weeks prior to the first dosing. For

subjects receiving prophylactic antimicrobial treatment (such as for prevention of urinary tract infection or chronic obstructive pulmonary disease exacerbation), they may be qualified to be enrolled after discussion with the sponsor.

17) Known human immunodeficiency virus (HIV) infection.

18) Having active hepatitis B virus (HBV) or hepatitis C virus (HCV) infection. Active HBV is defined as being positive for hepatitis B core antibody (HBcAb) or hepatitis B surface antigen (HBsAg), and the HBV deoxyribonucleic acid (DNA) level >200 IU/ml or 1000 copies/ml; subjects with HBV infection should receive antiviral treatment according to local treatment guidelines and be willing to receive antiviral treatment throughout the study period; active HCV is defined as being positive for hepatitis C antibody, and the HCV ribonucleic acid (RNA) level is greater than the ULN of the study center.

19) Any other primary malignancy within 3 years prior to the first dosing of the trial drugs, except for adequately resected non-melanoma skin cancer, cured carcinoma in situ, or other cured solid tumors.

20) Non-remission of toxicity of previous anticancer treatment, defined as toxicity not resolved to NCI CTCAE ≤1 grade (excluding alopecia and hyperpigmentation) or the level specified in the inclusion/exclusion criteria. For subjects with chronic grade 2 toxicity, if they are asymptomatic or can be adequately controlled with stable medications, they may be qualified to be enrolled after discussion with the sponsor.

21) History of severe hypersensitivity reactions to active pharmaceutical ingredients, inactive ingredients in preparations, or other monoclonal antibodies.

22) Lactating women, or women confirmed to be pregnant by pregnancy testing within 3 days prior to the first dosing.

23) The presence of any disease, medical condition, organ system dysfunction, or social situation, including but not limited to mental illness or substance/alcohol abuse, in the opinion of the investigator that may interfere with the subject's ability to provide informed consent, adversely affect the subject's ability to cooperate and participate in the study, or affect the interpretation of study results.

### III. Dosing Regimen

Subjects meeting all inclusion criteria and not meeting any of the exclusion criteria received the monotherapy of the anti-B7H3 antibody-drug conjugate (1) (lyophilized injection prepared according to conventional methods in this field) (with a dosage of 2.0 mg/kg or 2.4 mg/kg, intravenous infusion, once every three weeks as a cycle), the efficacy was evaluated every 6 weeks in the first 24 weeks, and then every 9 weeks until disease progression, intolerable toxicity, or voluntary withdrawal occurs.The study endpoint criteria include safety, ORR, DCR, pharmacokinetics, immunogenicity, and the like.

### IV. Therapeutic Effect

A total of 90 subjects were enrolled in the clinical study, including 68 cases of nasopharyngeal carcinoma (NPC) and 22 cases of lymphoepithelioma-like carcinoma (LELC). Among 63 NPC subjects with evaluable therapeutic effects, 60 subjects showed different degrees of tumor shrinkage, of which 32 subjects achieved objective response, the objective response rate (ORR) was 50.8%, and the disease control rate (DCR) was 95.2%. Among 19 LELC subjects with evaluable therapeutic effects, 17 subjects showed different degrees of tumor shrinkage, of which 9 subjects achieved objective response, the objective response rate (ORR) was 47.4%, and the disease control rate (DCR) was 89.5%.

Although the specific implementations of the present invention have been described above, those skilled in the art should understand that these are merely illustrative examples and that various changes or modifications may be made to these implementations without departing from the principles and essence of the present invention. Therefore, the scope of protection of the present invention is defined by the appended claims.

## Claims

1. A use of an anti-B7H3 antibody-drug conjugate, a pharmaceutically acceptable salt, stereoisomer, or metabolite thereof, or a solvate of any of the aforementioned items in the preparation of a drug for treating nasopharyngeal carcinoma and/or lymphoepithelioma-like carcinoma, the anti-B7H3 antibody-drug conjugate being of a structure of Formula I as follows,

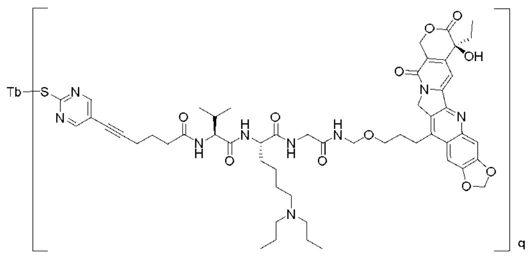

Formula I

wherein:

Tb is an anti-B7H3 antibody or an antigen-binding fragment thereof;
S is a sulfur atom on Tb;
q is a drug-antibody conjugate ratio, and is selected from any numerical value between 0.1 and 16.0.

**2.** The use according to claim 1, wherein the anti-B7H3 antibody or the antigen-binding fragment thereof comprises a heavy chain variable region and a light chain variable region; the heavy chain variable region comprises amino acid sequences CDR-H1, CDR-H2, and CDR-H3 as set forth in SEQ ID NOs: 1-3, respectively; the light chain variable region comprises amino acid sequences CDR-L1, CDR-L2, and CDR-L3 as set forth in SEQ ID NOs: 4-6, respectively, wherein CDRs are defined according to the Kabat numbering system;

preferably, the heavy chain variable region comprises an amino acid sequence as set forth in SEQ ID NO: 7 or having at least 85% sequence identity to SEQ ID NO: 7, and the light chain variable region comprises an amino acid sequence as set forth in SEQ ID NO: 8 or having at least 85% sequence identity to SEQ ID NO: 8;
more preferably, the anti-B7H3 antibody comprises a heavy chain and a light chain, wherein:
the heavy chain has an amino acid sequence as set forth in SEQ ID NO: 9 or having at least 85% sequence identity to SEQ ID NO: 9, and the light chain has an amino acid sequence as set forth in SEQ ID NO: 10 or having at least 85% sequence identity to SEQ ID NO: 10.

**3.** The use according to claim 1, wherein the q is selected from any numerical value between 1 and 10; preferably, q is 1, 2, 3, 4, 5, 6, 7, or 8; more preferably, q is 2, 4, 6, or 8.

**4.** The use according to claim 1, wherein the anti-B7H3 antibody-drug conjugate is an antibody-drug conjugate as shown in Formula II, a pharmaceutically acceptable salt, stereoisomer, or metabolite thereof, or a solvate of any of the aforementioned items,

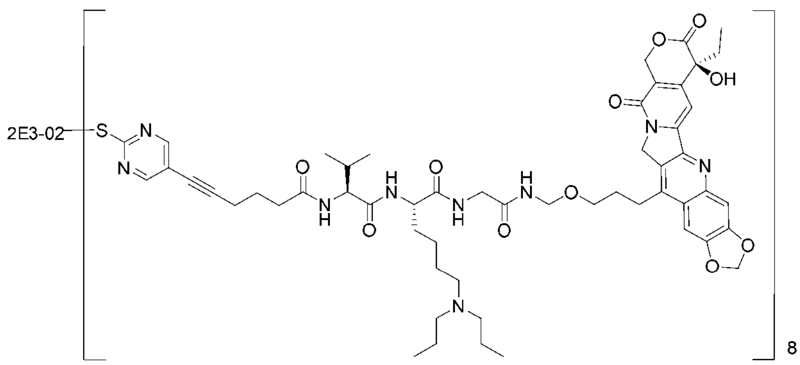

Formula II.

**5.** The use according to any one of claims 1-4, wherein the nasopharyngeal carcinoma is keratinizing squamous cell carcinoma.

**6.** The use according to any one of claims 1-4, wherein the nasopharyngeal carcinoma is non-keratinizing nasopharyngeal carcinoma, including a differentiated type and an undifferentiated type.

7. The use according to any one of claims 1-4, wherein the nasopharyngeal carcinoma is basaloid squamous cell carcinoma.

8. The use according to any one of claims 1-4, wherein the nasopharyngeal carcinoma is in situ nasopharyngeal carcinoma or infiltrative nasopharyngeal carcinoma, and the infiltrative nasopharyngeal carcinoma is preferably selected from squamous cell carcinoma, adenocarcinoma, microinvasive carcinoma, vesicular cell carcinoma, and undifferentiated nasopharyngeal carcinoma.

9. The use according to any one of claims 1-4, wherein the nasopharyngeal carcinoma is recurrent and/or advanced and/or metastatic nasopharyngeal carcinoma.

10. The use according to any one of claims 1-4, wherein the nasopharyngeal carcinoma and/or lymphoepithelioma-like carcinoma is not surgically resectable; or a patient with the nasopharyngeal carcinoma and/or lymphoepithelioma-like carcinoma has previously received chemotherapy and/or monoclonal antibody treatment and/or radiotherapy; or a patient with the nasopharyngeal carcinoma and/or lymphoepithelioma-like carcinoma has disease progression after previously receiving chemotherapy and/or monoclonal antibody treatment and/or radiotherapy.

11. The use according to claim 9, wherein a patient with metastatic or recurrent nasopharyngeal carcinoma refers to a patient who has previously received platinum-containing chemotherapy treatment and has recorded disease progression or intolerance during or after the treatment;

preferably, the patient with the metastatic or recurrent nasopharyngeal carcinoma refers to a patient who has previously received at least second-line (at least platinum-containing) chemotherapy treatment and has recorded disease progression or intolerance during or after the treatment;
preferably, the patient with the metastatic or recurrent nasopharyngeal carcinoma refers to a patient who has previously received at least second-line (at least first-line platinum-containing) chemotherapy treatment and has recorded disease progression or intolerance during or after the treatment.

12. The use according to claim 9, wherein a patient with metastatic or recurrent nasopharyngeal carcinoma refers to a patient who has previously received anti-PD-1 drug and/or anti-PD-L1 drug treatment and has recorded disease progression or intolerance during or after the treatment;

preferably, the patient with the metastatic or recurrent nasopharyngeal carcinoma refers to a patient who has previously received anti-PD-1 drug treatment and has recorded disease progression or intolerance during or after the treatment;
preferably, the patient with the metastatic or recurrent nasopharyngeal carcinoma refers to a patient who has previously received anti-PD-L1 drug treatment and has recorded disease progression or intolerance during or after the treatment.

13. The use according to claim 9, wherein a patient with metastatic or recurrent nasopharyngeal carcinoma refers to a patient who has previously received at least platinum-containing chemotherapy, anti-PD-1 drug and/or anti-PD-L1 drug treatment and has recorded disease progression or intolerance during or after the treatment;

preferably, the patient with the metastatic or recurrent nasopharyngeal carcinoma refers to a patient who has previously received platinum-containing chemotherapy and anti-PD-1 drug treatment and has recorded disease progression or intolerance during or after the treatment;
preferably, the patient with the metastatic or recurrent nasopharyngeal carcinoma refers to a patient who has previously received platinum-containing chemotherapy and anti-PD-L1 drug treatment and has recorded disease progression or intolerance during or after the treatment.

14. The use according to any one of claims 1-13, wherein the patient with the nasopharyngeal carcinoma and/or lymphoepithelioma-like carcinoma has previously received one or more of the following chemotherapeutic agents, including gemcitabine, capecitabine, cisplatin, lobaplatin, nedaplatin, fluorouracil, paclitaxel, albumin-bound paclitaxel, docetaxel, vinorelbine, cyclophosphamide, and pemetrexed.

15. The use according to any one of claims 1-14, wherein a patient with the nasopharyngeal carcinoma has previously received monoclonal antibody therapeutic agents, including camrelizumab, treprinumab, nivolumab, pembrolizumab, serplulimab, tislelizumab, cetuximab, or bevacizumab.

16. The use according to any one of claims 1-14, wherein the treatment comprises administering to the patient a therapeutically effective amount of an anti-B7H3 antibody-drug conjugate (preferably, Formula II), a pharmaceutically acceptable salt, stereoisomer, or metabolite thereof, or a solvate of any of the aforementioned items, wherein the effective amount is administered at a dose of 0.1-15 mg/kg body weight of an individual, and the dose is preferably 0.1-10 mg/kg, 0.2-8 mg/kg, 0.3-6 mg/kg, 0.4-4 mg/kg, 0.5-3.6 mg/kg, or 1-3 mg/kg, more preferably 0.4 mg/kg, 0.5 mg/kg, 0.6 mg/kg, 0.8 mg/kg, 1.0 mg/kg, 1.2 mg/kg, 1.4 mg/kg, 1.6 mg/kg, 1.8 mg/kg, 2.0 mg/kg, 2.1 mg/kg, 2.2 mg/kg, 2.3 mg/kg, 2.4 mg/kg, 2.5 mg/kg, 2.6 mg/kg, 2.7 mg/kg, 2.8 mg/kg, 2.9 mg/kg, 3.0 mg/kg, 3.2 mg/kg, 3.4 mg/kg, 3.6 mg/kg, 3.8 mg/kg, 4.0 mg/kg, or 5.0 mg/kg.

17. The use according to any one of claims 1-14, wherein the treatment comprises administering to the patient a therapeutically effective amount of an anti-B7H3 antibody-drug conjugate (preferably, Formula II), a pharmaceutically acceptable salt, stereoisomer, or metabolite thereof, or a solvate of any of the aforementioned items, wherein routes of administration thereof comprise oral administration, parenteral administration, and transdermal administration; the parenteral administration is preferably intravenous injection, subcutaneous injection, or intramuscular injection.

18. The use according to claim 17, wherein the route of administration is intravenous injection, wherein an injection form is an injection solution or a lyophilized powder, which contains an anti-B7H3 antibody-drug conjugate (preferably, Formula II), a pharmaceutically acceptable salt, stereoisomer, or metabolite thereof, or a solvate of any of the aforementioned items, and optionally a buffer, a stabilizer, a pH adjusting agent, and/or a surfactant, wherein the buffer may be selected from one or more of acetate, citrate, succinate, and phosphate; the stabilizer may be selected from sugar or amino acid, preferably disaccharide, such as sucrose, lactose, trehalose, or maltose; the pH adjusting agent may be selected from one or more of sodium hydroxide, lithium hydroxide, and potassium hydroxide; the surfactant may be selected from one or more of polyoxyethylene hydrogenated castor oil, glycerol fatty acid ester, and polyoxyethylene sorbitan fatty acid ester; the polyoxyethylene sorbitan fatty acid ester is preferably one or more of polysorbate 20, 40, 60, or 80, most preferably polysorbate 20.

19. The use according to any one of claims 1-14, wherein the treatment comprises administering to the patient a therapeutically effective amount of an anti-B7H3 antibody-drug conjugate (preferably, Formula II), a pharmaceutically acceptable salt, stereoisomer, or metabolite thereof, or a solvate of any of the aforementioned items, wherein the treatment comprises one or both of the following characteristics:

1) a dosing frequency is once daily, once weekly, once every two weeks, once every three weeks, once every four weeks or once monthly, once every five weeks, once every six weeks, or twice every two weeks, twice every three weeks, twice every four weeks or twice monthly, twice every five weeks, ortwice every six weeks; preferably once every three weeks or twice every three weeks;
2) a dosing cycle is one week, two weeks, three weeks, one month, two months, three months, four months, five months, six months, or longer; optionally, a duration of each dosing cycle is the same or different, and an interval between each dosing cycle is the same or different.

20. The use according to any one of claims 1-16, wherein the anti-B7H3 antibody-drug conjugate, the pharmaceutically acceptable salt, stereoisomer, or metabolite thereof, or the solvate of any of the aforementioned items is used as a sole active ingredient.

21. An anti-B7H3 antibody-drug conjugate, a pharmaceutically acceptable salt, stereoisomer, metabolite thereof, or a solvate for use in a method of treating cancer, wherein the method of treating cancer is by administering to a subject in need thereof with nasopharyngeal carcinoma and/or lymphoepithelioma-like carcinoma the anti-B7H3 antibody-drug conjugate, and the anti-B7H3 antibody-drug conjugate has a structural formula I:

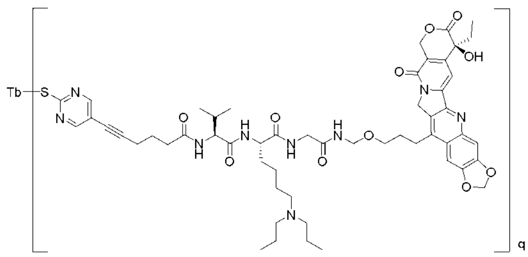

Formula I

wherein:

Tb is an anti-B7H3 antibody or an antigen-binding fragment thereof;
S is a sulfur atom on Tb;
q is any numerical value between 1 and 16, including both end values;
preferably, the anti-B7H3 antibody-drug conjugate is according to any one of claims 1-4.

**22.** A pharmaceutical kit, comprising (a) at least one unit dose of an anti-B7H3 antibody-drug conjugate, a pharmaceutically acceptable salt, stereoisomer, or metabolite thereof, or a solvate of any of the aforementioned items, and (b) instructions for use in the treatment of nasopharyngeal carcinoma and/or lymphoepithelioma-like carcinoma;
the anti-B7H3 antibody-drug conjugate is according to any one of claims 1-4, preferably Formula II.

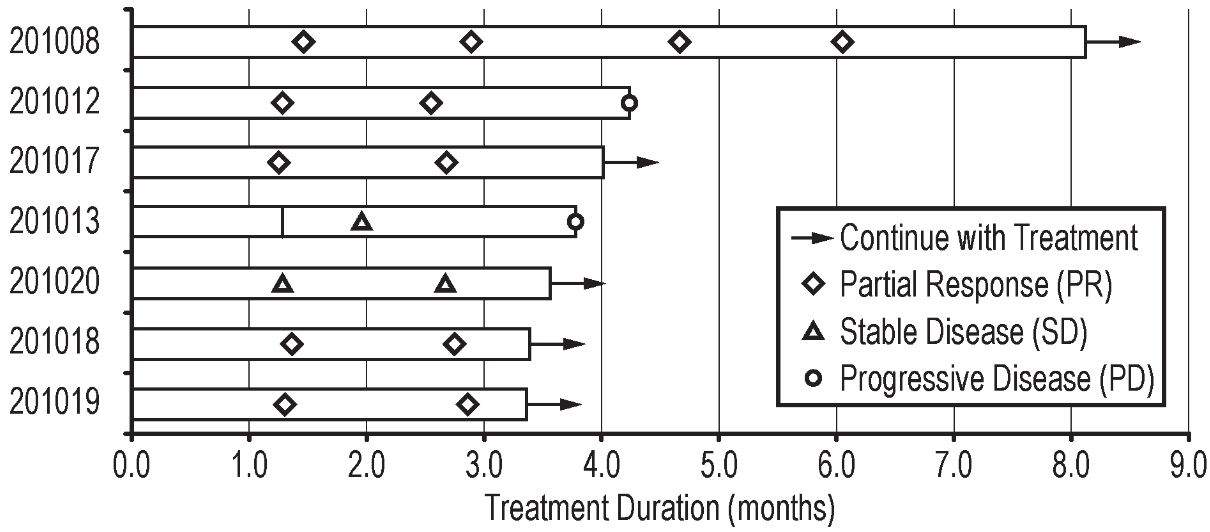
201008
201012
201017
201013
201020
201018
201019
Continue with Treatment
Partial Response (PR)
Stable Disease (SD)
Progressive Disease (PD)
0.0
1.0
2.0
3.0
4.0
5.0
6.0
7.0
8.0
9.0
Treatment Duration (months)

FIG. 1

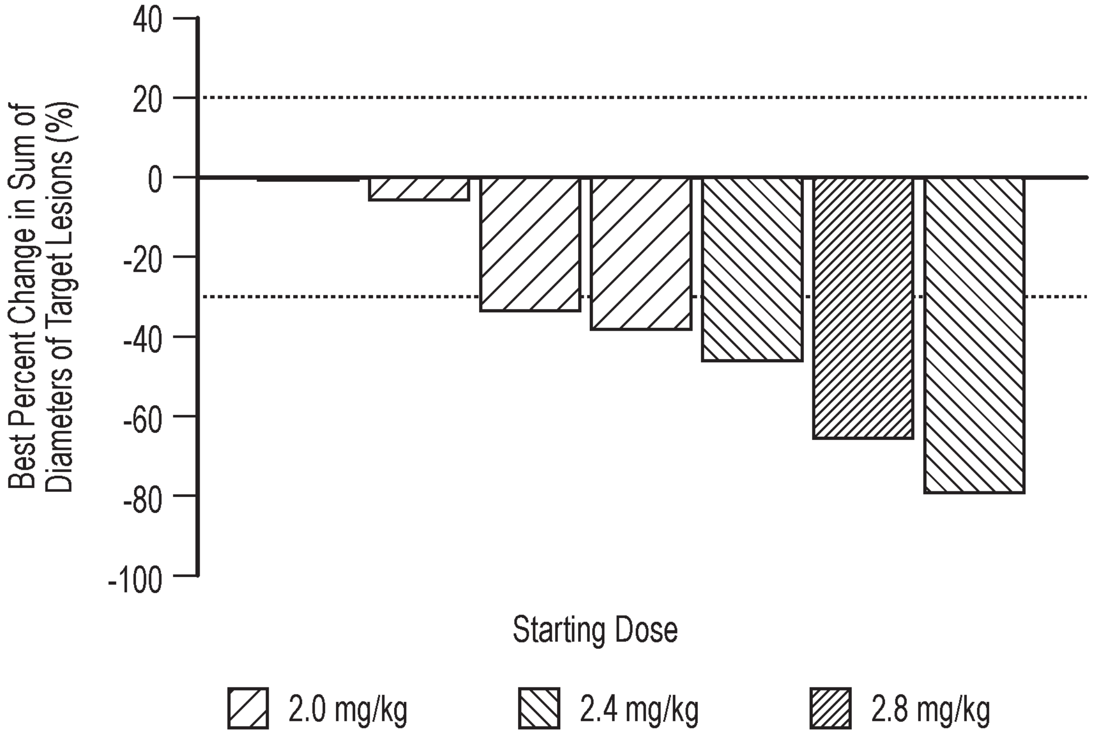
Best Percent Change in Sum of Diameters of Target Lesions (%)
40
20
0
-20
-40
-60
-80
-100
Starting Dose
2.0 mg/kg
2.4 mg/kg
2.8 mg/kg

FIG. 2

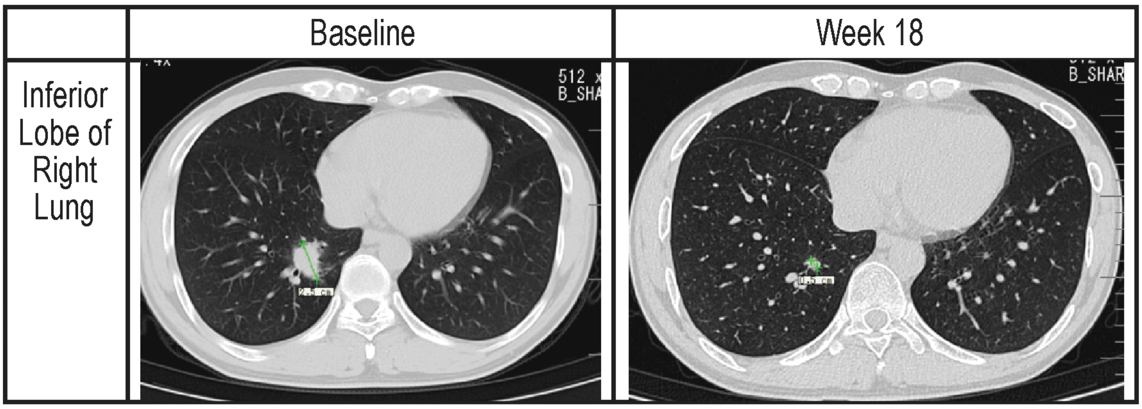
Baseline
Week 18
Inferior Lobe of Right Lung

FIG. 3

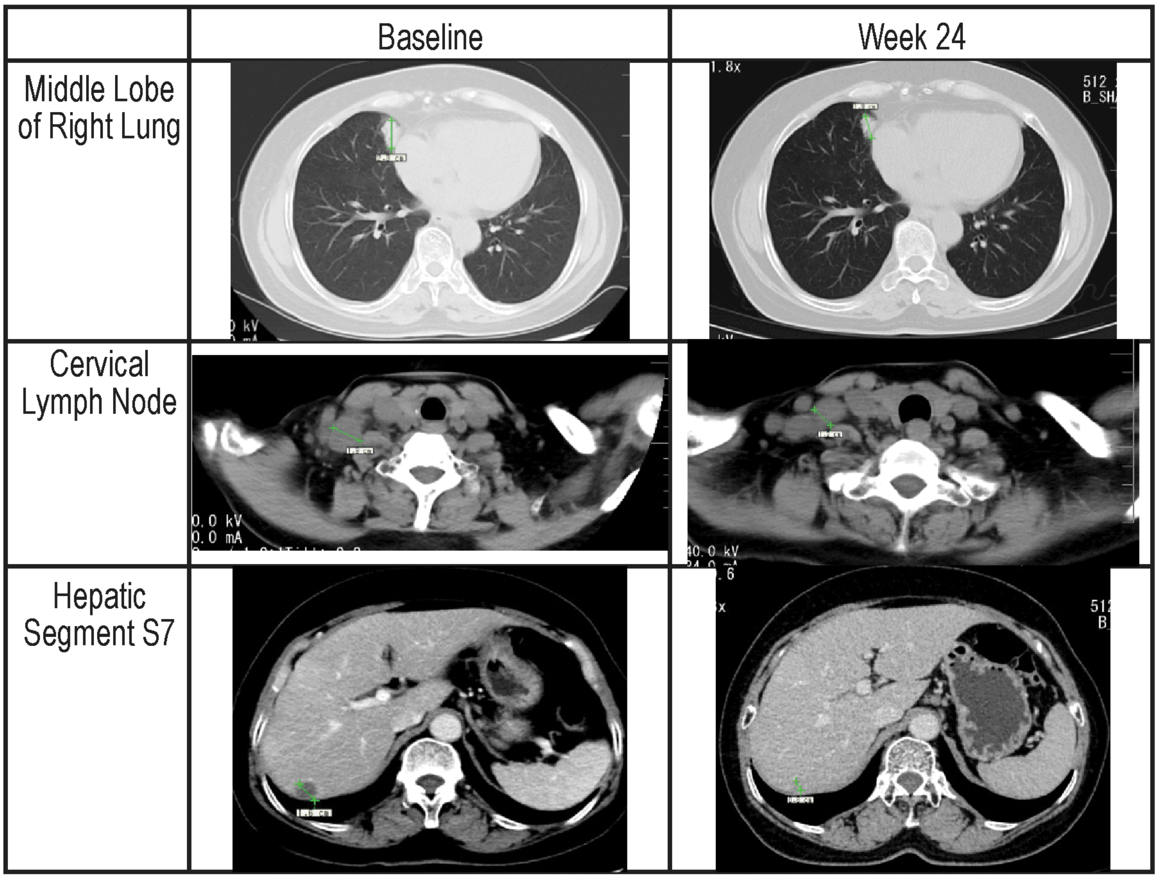
Baseline
Week 24
Middle Lobe of Right Lung
Cervical Lymph Node
Hepatic Segment S7

FIG. 4

**INTERNATIONAL SEARCH REPORT**

International application No.
**PCT/CN2024/119856**

**A. CLASSIFICATION OF SUBJECT MATTER**

A61K 47/68(2017.01)i； A61K 39/00(2006.01)i； C07K 16/28(2006.01)i； A61P 35/00(2006.01)i

According to International Patent Classification (IPC) or to both national classification and IPC

**B. FIELDS SEARCHED**

Minimum documentation searched (classification system followed by classification symbols)

IPC： A61K C07K A61P

Documentation searched other than minimum documentation to the extent that such documents are included in the fields searched

Electronic data base consulted during the international search (name of data base and, where practicable, search terms used)

CNTXT; DWPI; ENTXT; PUBMED; ELSEVIER; NCBI; VCN; Web of Science; STNext; CJFD; CNKI; 万方, WANFANG; 读秀, DUXIU; 中国专利生物序列检索系统, China Patents Biological Sequence Search System: 偶联物, ADC, B7H3, Conjugate, B7H3, nasopharyngeal carcinoma, pulmonary lymphoepithelioma-like carcinoma, 鼻咽癌, 肺淋巴上皮瘤样癌, 权利要求中的序列检索, sequence search in claims

**C. DOCUMENTS CONSIDERED TO BE RELEVANT**

| Category* | Citation of document, with indication, where appropriate, of the relevant passages | Relevant to claim No. |
|---|---|---|
| X | WO 2022170971 A1 (MEDILINK THERAPEUTICS (SUZHOU) CO., LTD.) 18 August 2022 (2022-08-18)<br>description, page 22, paragraphs 12-13, page 81, paragraphs 7 and 8, page 169, paragraph 2, and page 171, paragraph 8 | 21 |
| Y | WO 2022170971 A1 (MEDILINK THERAPEUTICS (SUZHOU) CO., LTD.) 18 August 2022 (2022-08-18)<br>description, page 22, paragraphs 12-13, page 81, paragraphs 7 and 8, page 169, paragraph 2, and page 171, paragraph 8 | 1-20, 22 |
| Y | 汪春福#等 (WANG, Chunfu et al.). "鼻咽癌中B7H3的表达及其临床意义 (Expression of B7-H3 in Nasopharyngeal Non-Keratinizing Carcinoma and its Clinical Significance)" *Journal of Modern Oncology*, Vol. 17, No. 12, 31 December 2009 (2009-12-31), pages 2290-2292<br>page 2292, right-hand column, last paragraph | 1-20, 22 |

☑ Further documents are listed in the continuation of Box C. ☑ See patent family annex.

* Special categories of cited documents:
- "A" document defining the general state of the art which is not considered to be of particular relevance
- "D" document cited by the applicant in the international application
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **02 December 2024** | **09 December 2024** |

| Name and mailing address of the ISA/CN | Authorized officer |
|---|---|
| **China National Intellectual Property Administration (ISA/CN)**<br>**China No. 6, Xitucheng Road, Jimenqiao, Haidian District, Beijing 100088** | Telephone No. |

Form PCT/ISA/210 (second sheet) (July 2022)

**INTERNATIONAL SEARCH REPORT**

International application No.
**PCT/CN2024/119856**

**C. DOCUMENTS CONSIDERED TO BE RELEVANT**

| Category* | Citation of document, with indication, where appropriate, of the relevant passages | Relevant to claim No. |
|---|---|---|
| Y | 洪少东 (HONG, Shaodong). "长在肺里的“鼻咽癌”基因图谱被中肿医学科学家解密 (Non-official translation: The Genetic Map of "Nasopharyngeal Carcinoma in the Lung" Deciphered by Medical Scientists from Sun Yat-sen University Cancer Center)" *https://www.sysucc.org.cn/node/2787,* 18 July 2009 (2009-07-18), pages 2-5<br>page 4, section 03 | 1-20, 22 |

**INTERNATIONAL SEARCH REPORT**

International application No.
**PCT/CN2024/119856**

**Box No. I Nucleotide and/or amino acid sequence(s) (Continuation of item 1.c of the first sheet)**

1. With regard to any nucleotide and/or amino acid sequence disclosed in the international application, the international search was carried out on the basis of a sequence listing:
   a. ☑ forming part of the international application as filed.
   b. ☐ furnished subsequent to the international filing date for the purposes of international search (Rule 13*ter*.1(a)),
      ☐ accompanied by a statement to the effect that the sequence listing does not go beyond the disclosure in the international application as filed.

2. ☐ With regard to any nucleotide and/or amino acid sequence disclosed in the international application, this report has been established to the extent that a meaningful search could be carried out without a WIPO Standard ST.26 compliant sequence listing.

3. Additional comments:

Form PCT/ISA/210 (continuation of first sheet) (July 2022)

**INTERNATIONAL SEARCH REPORT**
**Information on patent family members**

International application No.
**PCT/CN2024/119856**

| Patent document cited in search report | Publication date (day/month/year) | Patent family member(s) | Publication date (day/month/year) |
|---|---|---|---|
| WO 2022170971 A1 | 18 August 2022 | CA 3206117 A1 | 18 August 2022 |
| | | MX 2023008895 A | 09 August 2023 |
| | | AU 2022220512 A1 | 13 July 2023 |
| | | AU 2022220512 A9 | 24 October 2024 |
| | | KR 20230145038 A | 17 October 2023 |
| | | JP 2024508081 A | 22 February 2024 |
| | | EP 4257154 A1 | 11 October 2023 |
| | | IL 304804 A | 01 September 2023 |
| | | US 2024108745 A1 | 04 April 2024 |
| | | TW 202241521 A | 01 November 2022 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- CN 202311206601
- CN 202411170320
- WO 2022170971 A
- US 4816567 A
- WO 2022170971 A1
- WO 2023143208 A1


### Non-patent literature cited in the description


- **WANG YQ et al.** Development and validation of an immune checkpoint-based signature to predict prognosis in nasopharyngeal carcinoma using computational pathology analysis. *J Immunother Cancer.*, 13 November 2019, vol. 7 (1), 298
- **MORRISON et al.** *PNAS*, 1984, vol. 81, 6851-6855
- **E. A. EISENHAUER et al.** *EUROPEAN JOURNAL OF CANCER*, 2009, vol. 45, 228-247